# EUROPEAN PATENT SPECIFICATION

(11) **EP 4 479 550 B1**
(45) Date of publication and mention of the grant of the patent: **27.05.2026**
(21) Application number: 23711368.3
(22) Date of filing: 16.02.2023
(51) Int. Cl.: C12Q 1/6804, G01N 33/543

(54) **METHODS FOR PROTEIN DETECTION**
VERFAHREN ZUM PROTEINNACHWEIS
PROCÉDÉS DE DÉTECTION DE PROTÉINES

(30) Priority: 17.02.2022 US 202263268136 P
(43) Date of publication of application: 25.12.2024
(73) Proprietor: One Lambda, Inc., West Hills, California 91304 (US); Life Technologies Corporation, Carlsbad, CA 92008 (US)
(72) Inventor: PEI, Rui, West Hills, California 91304 (US); VANDER HORN, Peter B., Carlsbad, California 92008 (US); MA, Hong, West Hills, California 91304 (US); WAKEFIELD, Ito, West Hills, California 91304 (US)
(74) Representative: Boult Wade Tennant LLP
(86) International application number: PCT/US2023/062740
(87) International publication number: WO 2023/159127

(56) References cited:
- WO-A2-2012/145725
- US-A1- 2014 274 775
- WU WEI ET AL: "A sensitive lateral flow biosensor forEscherichia coliO157:H7 detection based on aptamer mediated strand displacement amplification", ANALYTICA CHIMICA ACTA, ELSEVIER, AMSTERDAM, NL, vol. 861, 24 December 2014 (2014-12-24), pages 62 - 68, XP029198888, ISSN: 0003-2670, DOI: 10.1016/J.ACA.2014.12.041
- FANG ZHIYUAN ET AL: "Lateral flow biosensor for DNA extraction-free detection of salmonella based on aptamer mediated strand displacement amplification", BIOSENSORS AND BIOELECTRONICS, vol. 56, 1 June 2014 (2014-06-01), Amsterdam , NL, pages 192 - 197, XP093047581, ISSN: 0956-5663, DOI: 10.1016/j.bios.2014.01.015
- WANG PENGZHI ET AL: "Proximity ligation assay: an ultrasensitive method for protein quantification and its applications in pathogen detection", APPLIED MICROBIOLOGY AND BIOTECHNOLOGY, vol. 105, no. 3, 11 January 2021 (2021-01-11), pages 923 - 935, XP037350664, ISSN: 0175-7598, DOI: 10.1007/S00253-020-11049-1
- CHRISTINA GREENWOOD ET AL: "Proximity assays for sensitive quantification of proteins", BIOMOLECULAR DETECTION AND QUANTIFICATION, vol. 4, 1 June 2015 (2015-06-01), pages 10 - 16, XP055364680, ISSN: 2214-7535, DOI: 10.1016/j.bdq.2015.04.002
- KRIS JANSSEN ET AL: "Nucleic Acids for Ultra-Sensitive Protein Detection", SENSORS, vol. 13, no. 1, 21 January 2013 (2013-01-21), pages 1353 - 1384, XP055223466, DOI: 10.3390/s130101353

## Description

### CROSS REFERENCE TO RELATED APPLICATIONS

This application claims benefit of U.S. Provisional Application No. 63/268,136 filed February 17, 2022.

### TECHNICAL FIELD

Aspects of the present disclosure relate methods, devices, and system for detection of analyte in a biological sample, which analyte can include a target protein having a low concentration in the sample. Other aspects relate to methods, devices, and systems for such detection that are suitable for point-of-care and other single-use applications.

### INTRODUCTION

Detection of an analyte such as a target protein present in a low amount, whether due to overall sample size or otherwise low concentration in a sample, poses challenging problems. For example, in some cases, intrinsic concentration of an analyte in a sample can fail to yield a product suitable for diagnostic processing. Further, processing the sample to achieve a suitable concentration for detection may require relatively laborious procedures. Moreover, sample volume may be limited so as to prohibit consideration of laborious sample concentration procedures.

In addition, because of the low sample volumes generally associated with "point-of-care" techniques, protein detection poses even greater challenges due to the sensitivities that may be needed to achieve accurate diagnostic indications. For example, use of lateral flow substrates and technologies to bind target proteins has faced difficulty in achieving the sensitivities needed for effective and accurate detection. Wang Pengzhi et al. Appl Micobiol and Biotechnol (2021) 105(3):923-935 summarizes the basic principles of PLA (proximity ligation assay) and its applications in pathogen detection.

Accordingly, a need exists for methods, systems, and devices that can solve or otherwise ameliorate such problems associated with detection of proteins.

### SUMMARY OF THE INVENTION

The invention is defined by appendant claims.

### BRIEF DESCRIPTION OF THE DRAWINGS

The accompanying drawings, which are incorporated into and form a part of the specification, illustrate embodiments of the present disclosure and, together with the description, serve to explain the principles of the disclosure. The drawings are only for the purpose of illustrating some embodiments of the disclosure and are not to be construed as limiting the disclosure. In the drawings:
FIG. 1 is a work flow process for performing a protein detection assay in accordance with various embodiments of the present disclosure.
FIG. 2A through FIG. 2C depict schematic diagrams of stages of a proximity assay in accordance with various embodiments the present disclosure.
FIG. 3A is a graph schematically illustrating a series of hypothetical curves of fluorescence v. cycle number for differing analyte concentrations.
FIG. 3B is a graph schematically illustrating a hypothetical standard curve of cycle number v. target analyte concentration as can be derived from results depicted in FIG. 3A.
FIG. 4 is a schematic diagram of oligonucleotides and their joined relationships to form a single strand of template nucleic acid from a proximity ligation assay in accordance with various embodiments of the present disclosure.
FIG. 5 is a schematic representation of components for labeling and detection of a proximity oligonucleotide product using detection and capture probes of a lateral flow device of the present disclosure.
FIG. 6 is a schematic representation of a lateral flow substrate for detection in accordance with various embodiments of the present disclosure.
FIG. 7 is a plan view of an embodiment of a fluidic component of a fluidic device that can be utilized for performing a protein detection assay of the present disclosure.
FIG. 8A and FIG. 8B are schematic representations of a vent pocket of a fluidic device of the present disclosure in accordance with various embodiments of the present disclosure, showing a (closed) sealed and open (unsealed) state respectively.
FIG. 8C illustrates an exploded view of a fluidic component of a fluidic device, such as the fluidic component of FIG. 7, in accordance with various embodiments of the present disclosure.
FIG. 9 depicts various views illustrating resistive heater details in a fluidic device in accordance with various embodiments of the present disclosure.
FIG. 10 depicts various views schematically illustrating a gas expansion technique of transporting fluid through a fluidic device in accordance with various embodiments of the present disclosure.
FIGS. 11 and 12 depict various views illustrating the detail and function of labeling in a fluidic device in accordance with various embodiments of the present disclosure.
FIG. 13 depicts a plan view of an embodiment of a fluidic device utilizing a fluidic component, in accordance with various embodiments of the present disclosure.
FIG. 14 depicts the device of FIG. 13 in place in a docking unit in accordance with the present disclosure.
FIG. 15 is a perspective view of an embodiment of a fluidic device that can be utilized for performing a protein detection assay of the present disclosure.
FIG. 16 is a plan view of an embodiment of a fluidic component of the present disclosure, such as can be used with fluidic device FIG. 15.
FIG. 17 s a plan view of an embodiment of a fluidic component of the present disclosure, such as can be used with fluidic device FIG. 15.
FIG. 18 is an expanded view of an exemplary reagent recess of the present disclosure.
FIG. 19 is a view of an exemplary chamber with a triangular flow control feature projecting into a chamber in a first orientation.
FIG. 20 is a view of an exemplary chamber with a triangular flow control feature projecting into a chamber in a second orientation.
FIG. 21A and FIG. 21B are schematic representations illustrating how vent pocket opening to an internal volume provides fluidic flow control in a hermetically-sealed fluidic device.
FIG. 22 depicts an exemplary vent pocket of the present disclosure comprising a dimple structure.
FIG. 23 is an embodiment of a flexible circuit comprising resistive heating elements corresponding to various chambers and vent pockets of fluidic devices of the present disclosure
FIG. 24 is an exploded view of an embodiment of a fluidic device of the present disclosure.
FIG. 25 depicts a front perspective view of a docking unit of the present disclosure shown with the lid in the open position and a fluidic device inserted.
FIG. 26 depicts a front perspective view of the interior of a docking unit providing a display of infrared temperature sensors used to monitor the temperature of various processing chambers.
FIG. 27 depicts a back perspective view of the interior of a docking unit providing a display of infrared temperature sensors used to monitor the temperature of various processing chambers.

### DETAILED DESCRIPTION

### PROTEIN ANALYSIS IN LATERAL FLOW DEVICES AND SYSTEMS

Various embodiments in accordance with the present disclosure pertain to methods, systems, and devices for detection of a target analyte, for example, a target protein in a sample. More specifically, methods, systems, and devices are disclosed that provide relatively high sensitivity to such detection so as to be able to determine the presence of the target analyte at relatively low concentrations and/or in relatively small sample volumes. Various embodiments of the present disclosure enable the use of a lateral flow substrate for detection of target protein in a sample. The ability to achieve such detection is unexpected in view of the difficulty heretofore encountered in being able to achieve sensitivities high enough to achieve such detection. To achieve such sensitivity when using a lateral flow substrate for detection, embodiments in accordance with the present disclosure utilize a target-linked template nucleic acid generation technique, such as occurs in a proximity assay, to first generate a template nucleic acid from a complex generated from the target protein of interest and amplify the template nucleic acid for subsequent detection on a lateral flow substrate. In this way, detection of the template nucleic acid serves as a proxy for detection of the target protein and can confirm the presence of the initial protein in the sample.

The terms "analyte" is to be construed broadly as any compound, molecule, or other substance of interest to be detected, identified, or characterized. For example, the analyte can include, but is not limited to, at least one of amino acids, nucleic acids, polypeptides, proteins, polynucleotides, lipids, phospholipids, sugars, polysaccharides, and combinations thereof. Specific examples of analyte can include proteins, metabolites, allergens, hormones, toxins, RNA (e.g., mRNA, total RNA, tRNA, etc.), DNA (e.g., plasmid DNA, plant DNA, etc.), tagged proteins, antibodies, antigens, and combinations thereof, but are not limited to these.

The terms "polypeptide", "peptide", and "protein" are used interchangeably herein to designate a linear series of amino acid residues connected one to the other by peptide bonds, which includes proteins, polypeptides, oligopeptides, peptides, and fragments thereof. The protein may be made up of naturally occurring amino acids and/or synthetic (e.g. modified or non-naturally occurring) amino acids. Thus "amino acid", or "peptide residue", as used herein means both naturally occurring and synthetic amino acids. The terms "polypeptide", "peptide", and "protein" includes fusion proteins, including, but not limited to, fusion proteins with a heterologous amino acid sequence, fusions with heterologous and homologous leader sequences; immunologically tagged proteins; fusion proteins with detectable fusion partners, e.g., fusion proteins including as a fusion partner a fluorescent protein, β-galactosidase, luciferase, etc.; and the like.

Aside from the use of lateral flow substrate technology for detection of target protein in relatively small initial starting amounts, various embodiments of the present disclosure further enable detection via other mechanisms, such as via use of other substrates for binding the amplified template nucleic acid (whether or not the substrate is a lateral flow substrate) and/or the use of forms of detectable substances other than those producing colorimetric indicators, such as fluorescence for example. Regardless of the other mechanisms, however, the ability to achieve higher sensitivities in detection may enable protein detection using what is referred to as "point-of-care" technology, as the sample amount and processing in accordance with various embodiments of the present disclosure is reduced and simplified and able to be carried out in self-contained fluidic devices that do not require multiple fluid handling and processing steps by a user; rather, such devices permit much of the fluidic flow control and processing to occur within a single fluidic network of a fluidic device, which can occur automatically once sample has been introduced to the device. Moreover, various embodiments of the present disclosure enable the final result of the assay to detect the presence of the target protein to occur within relatively short timeframes, compatible with point-of-care applications. Further, the present disclosure is not limited to point-of-care applications and can be implemented in any environments or settings where detection on one or more target analytes of interest is desired.

Various embodiments can permit use of a system that utilizing a lateral flow substrate for detection, for example combined with a fluidic device in a single disposable device. For example, a fluidic device comprising, but not limited to, a housing, an electronic layer (comprising a printed circuit board assembly), and a fluidic layer is provided. By way of non-limiting example, fluidic devices can be used or modified as described in WO/2012/145730, "Integrated Device for Nucleic Acid Detection and Identification,". A system utilizing such a device can utilize a reusable docking unit to program instructions for carrying out various temperature and flow control on the fluidic device to support the various reactions of a protein detection assay, and also to provide power and a user interface, enabling the use of a relatively simplified fluidic device that can be suitable for single use applications.

Fluidic devices in accordance with various embodiments can include reagents that can be in or proximal to a chamber in which sample processing occurs. Such reagents can be predisposed lyophilized reagents, which further assists in reducing overall sample and/or reagent handling steps, and better adapting the fluidic devices to single-use and/or point-of-care applications. For example, a first chamber or a recess proximal to the first chamber, can include a lyophilized powder, pellet, or film derived from a formulation of a first proximity probe, a second proximity probe, and an oligonucleotide splint in suitable buffers, salts and stabilizers. As will be described in more detail herein, a proximity probe is a molecule formed, for example, through conjugation of an antibody or antibody fragment to an oligonucleotide. Additionally, a second chamber or a recess proximal to the second chamber, can include a lyophilized powder, pellet, or film derived from a formulation of deoxyribonucleotides, ribonucleotides, oligonucleotide primers, and enzymes such as DNA polymerase and reverse transcriptase, in suitable buffers, salts and stabilizers. Finally, lyophilized detection probes for labeling nucleic acid (including detection particles as further described herein), and/or capture probes and moieties, can be embedded in a lateral flow substrate or otherwise provided on structures or surfaces within the fluidic device. Such lyophilized reagents can be solubilized upon addition of the sample or other aqueous substance.

In various embodiments, a lateral flow substrate is used, for example, a colorimetric detection strip can be used to detect amplified nucleic acids. The use of porous materials to comprise the sample receiving zone and the labeling zone can result in the retention of some sample solution as well as detection particles in the porous materials. Although labeling zones comprising porous materials having reversibly immobilized moieties utilized for detection can be used, a device can utilize detection particles or moieties held in a region of the device distinct from the sample receiving zone of the lateral flow strip and comprising nonporous materials with low fluid retention characteristics. This approach allows amplified template nucleic acid to be labeled prior to introduction to the porous components of the sample receiving end of the lateral flow component of the device and thereby eliminates the retention and/or loss of sample material and detection probes in a porous labeling zone. This technique can further enable the use of various treatments of the sample in the presence of detection moieties, such as treatment with high temperatures, to accomplish denaturation of a double-stranded target or secondary structures within a single-stranded target without concern for the impacts of temperature on porous sample receiving or labeling zone materials or the lateral flow detection strip materials.

The use of a labeling zone not in lateral flow contact with the sample receiving zone but subject to the control of fluidic components such as vents or valves allows target and label to remain in contact for periods of time controlled by fluid flow control systems. By incorporating the detection probes in a temperature-regulated chamber, denaturation of duplex nucleic acid can be possible allowing for efficient hybridization-based detection. In addition, or in the alternative, fluorescence sensing can be used to detect amplified nucleic acid products. These optical detection systems can be configured to perform real-time nucleic acid detection and quantification during amplification and end-point detection after amplification.

Various embodiments enable detection of a target protein in the sample at a concentration of less than about 10 mg/mL, less than about 1.0 mg/mL, less than about 100 ng/mL, less than about 10 ng/mL, less than about 1.0 ng/mL, less than about 100 pg/mL (picograms/mL), less than about 10 pg/mL, or less than about 1.0 pg/mL, or any intervening range or amount. The target protein or other target analyte can be present in the sample at a concentration of less than about 10 mM, less than about 1.0 mM, less than about 100 nM, less than about 10 nM, less than about 1.0 nM, less than about 100 pM (picomolar), less than about 10 pM, less than about 1.0 pM, less than about 0.1 pM, less than about 0.01 pM, less than about 0.001 pM, or less than 0.0001 pM, or any intervening range or amount. Further, sample volumes in accordance with various embodiments can range from about 10 µL to about 70 µL.

The sample can be an unprocessed or pre-processed sample to ready it for subsequent reactions of the protein detection assay. The sample can comprise one or more biological samples, which can be in their original form, modified, or otherwise processed, for example lysing or other treatments can occur to extract proteins. For example, the biological fluid can be reconstituted. The sample can be from any source biological, or non-biological, or both. A biological sample can be from any organism, for example, a human, a primate, a mammal, a vertebrate, an invertebrate, a plant, a fungus, a bacterium, or a virus, or any combination thereof, and can be in the form of blood, plasma, tissue, urine, feces, saliva, semen, and other bodily fluids.

While use of a single fluidic device is contemplated to perform the various processing of the sample for template nucleic acid generation, amplification, labeling, and detection, various embodiments of the present disclosure contemplate that some of the workflow could occur in different devices or systems, as will be better appreciated from the description of various embodiments and figures that follows.

In accordance with various embodiments, processing the sample can include contacting it with one or more reagents configured for generating a template nucleic acid by utilization of a oligonucleotide conjugate having a specific affinity to bind to the target protein and generating the template nucleic acid as a result of linking such oligonucleotide conjugate to the target protein. Such target-linked template nucleic acid generation can be accomplished by using performing a proximity ligation assay (PLA), or a proximity extension assay (PEA), or both. Examples of proximity assays within the scope of the disclosure are described in Greenwood et al., "Proximity Assays for Sensitive Quantification of Proteins," Biomolecular Detection and Quantification, 4:10-16 (2015). The terms proximity reaction or proximity template nucleic acid generation, and permutations thereof, also may be used herein to refer to the portion of a proximity assay that yields the template nucleic acid. Processing of the sample may further include contacting the sample with one or more reagents configured for performing an amplification reaction to amplify template nucleic acid generated by the proximity reaction and subjecting the sample to amplification conditions, which can be isothermal amplification conditions or thermal cycling conditions. The one or more reagents configured to perform an amplification can comprise reagents for a polymerase chain reaction (PCR), or for a rolling circle amplification (RCA) assay, or both. Processing of a sample can additionally comprise contacting a sample with one or more enzymes, for example, a ligase, or a polymerase, or both.

In a sample in which a target protein is present, the processing above results in performing target-linked template nucleic acid generation, which is the process of linking a known oligonucleotide to a target protein through a moiety that has an affinity for binding to the target protein, and thereafter using the target-linked oligonucleotide in generate a template nucleic acid. Such target-linked template generation can occur by performing the template generation process involved in a proximity assay, such as PLA, PEA, or a combination of both. The terms proximity reaction, proximity template nucleic acid generation, and permutations thereof are used interchangeably herein to refer to the processing of a sample up through the template nucleic acid generation portion of a proximity assay, i.e., prior to the amplification of the template nucleic acid. After performing target-linked template nucleic acid generation, amplification processing of the sample results in an amplified product of the template nucleic acid (i.e., amplicons of the template nucleic acid). In various embodiments, the target-linked template generation can occur in a processing stage separate from the amplification reaction, but the two can be combined in a single stage as well, for example using a mixture of reagents and allowing the reactions to occur in the same chamber.

Proximity template generation to generate a template nucleic acid can comprise contacting the sample with one or more probes with affinity for a target analyte, for example, a target protein. The probe can comprise a first moiety with affinity for the target protein and a second moiety comprising an oligonucleotide configured for generating the template nucleic acid. The first and second moieties can be directly bound to one another or can be bound to each other through intermediate molecules, for example, streptavidin/avidin and biotin. In various embodiments, first and second probes are used and are proximity probes, with the first proximity probe having an affinity for a first epitope of the target protein and the second proximity probe having an affinity for a second epitope of the target protein. The first proximity probe can further comprise a first oligonucleotide and the second proximity probe can comprise a second oligonucleotide. Proximity template generation can further include hybridizing a third oligonucleotide to both the first and second oligonucleotides to form the template nucleic acid, which can be facilitated by one or more enzymes, for example, a ligase, or a polymerase, or both. The third oligonucleotide can be referred to as a "splint" oligonucleotide in that it splints and joins the free ends of the first and second oligonucleotides. The third oligonucleotide can be free or attached to a third proximity probe. The first and second proximity probes can have differing affinities to the target protein, for example, binding to different epitopes of the target protein.

The first proximity probe can further comprise a first antibody or a first antibody fragment, the first antibody or the first antibody fragment having affinity for the first epitope. The second proximity probe can comprise a second antibody or a second antibody fragment, the second antibody or the second antibody fragment having an affinity for the second epitope. The first oligonucleotide can be configured to be bound through a 5'-end to the first antibody or first antibody fragment. The second oligonucleotide can be configured to be bound through a 3'-end to the second antibody or second antibody fragment. The first oligonucleotide can be configured to be bound by its 5'-end to the first antibody or first antibody fragment through one or more intervening molecules. The second oligonucleotide can be bound by its 3'-end to the second antibody or second antibody fragment through one or more intervening molecules.

The first antibody or first antibody fragment can be a first primary antibody or a first primary antibody fragment. The second antibody or the second antibody fragment can be a second primary antibody or a second primary antibody fragment. The first proximity probe can further comprise a first secondary antibody or a first secondary antibody fragment. The first secondary antibody or the first secondary antibody fragment can have affinity for the first primary antibody or the first primary antibody fragment. The second proximity probe further can comprise a second secondary antibody or a second secondary antibody fragment. The second secondary antibody or the second secondary antibody fragment can have affinity for the second primary antibody or the second primary antibody fragment. The first oligonucleotide can be configured to be bound through a 5'-end to the first secondary antibody or first secondary antibody fragment. The second oligonucleotide can be configured to be bound through a 3'-end to the second secondary antibody or second secondary antibody fragment. An antibody or antibody fragment can be substituted with a DNA aptamer or other binding moiety with specificity for the respective target epitope.

The template nucleic acid ultimately generated from the initial single strand proximity template generation can comprise double-stranded DNA and the amplification can comprise the use of one or more amplification reagents, for example, a forward primer, a reverse primer, and a heat stable DNA polymerase. The forward primer can hybridize to a first strand of a double-stranded DNA template and the reverse primer can hybridize to a second strand of the double-stranded DNA template.

Additional processing of a sample in accordance with various embodiments includes contacting the product of the amplification reaction with one or more labeling reagents. In this manner, any amplified product (amplicons of the template nucleic acid) can be labeled, for example using a detection probe comprising a detectable label such as a colored bead, a fluorophore, or other suitable detectable label and a moiety having an affinity to bind to the amplicons of the template nucleic acid.

Additional processing can further include performing a detection for labeled product. In various embodiments, a capture moiety can be anchored to a substrate and the capture moiety is configured to bind labeled amplified template nucleic acid. The substrate can be a lateral flow substrate, such as any of a variety of porous matrix substrates that can wick for provide capillary flow of aqueous substances therethrough, and the detection can be by observation of a color formed on the substrate (colorimetric detection). The present disclosure is not so limited however and other types of capturing substrates are envisioned, such as providing capture moieties on walls of a fluidic chamber or channel, on a semi-solid gel or the like contained in a fluidic chamber or channel, or on beads or other particles in such chambers to anchor the labeled amplified template nucleic acid for later detection, which can be via a fluorescence sensing or other reader in the case of the detectable label being a fluorophore.

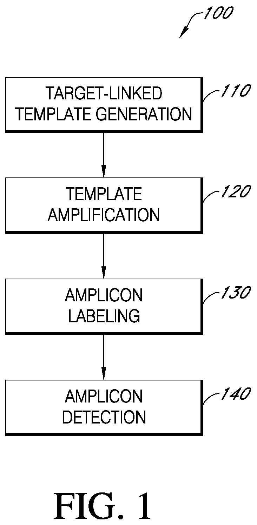

Turning to the figures, various embodiments of the present disclosure provide a greater understanding of the disclosure and principles of operation. FIG. 1 is a flow diagram depicting an embodiment of a workflow 100 for the detection of a target protein. At 110, target-linked template generation is performed on a biological sample. The template generation can occur via probes configured to be linked structurally and/or functionally to a target protein. In general, target-linked template generation is the formation of template nucleic acid based on the ability of the template nucleic acid to bind specifically to a target protein of interest, either through binding of an antibody or directly to the protein. Target-linked template generation can occur via any of a variety of reactions in which a template nucleic acid is produced via linkage to the target analyte (target protein) of interest. In various embodiments, proximity assay reactions can be used to perform the target-linked template generation, such reactions including, for example, the reactions to generate a template nucleic acid that occur in a proximity ligation assay (PLA) or a proximity extension assay (PEA). After target-linked template generation occurs, template nucleic acid amplification is performed on any of the template nucleic acid product that may be generated at 110. Thus, at 120, assuming the initial presence of the target protein and subsequent template nucleic acid generation at 110, amplification product (e.g., copy amplicons of the template nucleic acid) is generated at 120. Amplification can occur, for example, via polymerase chain reaction (PCR) or rolling circle amplification. Thermal cycling or isothermal amplification can be used.

After amplification of template nucleic acid has occurred to produce an amplification product, amplicon labeling occurs at 130. Here, the amplicons generated via amplification of template nucleic acid are individually labeled to produce a labeled product. For example, detection probes can be introduced to the amplification product to bind to the amplicons. Labeling may occur via probes attached to colored particles, such as beads, or via probes carrying fluorescent dye, for example. But such are not limited and additional detection probe embodiments for performing labeling of amplicon are envisioned as within the scope of the present disclosure.

Detection probe as used herein refers to a conjugate used to label nucleic acid product (amplicons) generated during an amplification reaction, and includes fluorescent dyes specific for duplex nucleic acid, dye-modified oligonucleotides, such as fluorescently-dye modified oligonucleotides, oligonucleotide-conjugated quantum dots, or oligonucleotide-conjugated solid phase elements such as a polystyrene, latex or paramagnetic particles or microspheres. As used herein, beads, particles and microspheres can be used interchangeably. Suitable detection probes include but are not limited to a range of dyes including visible dyes and fluorescent dyes specific for duplex nucleic acid, fluorescently modified oligonucleotides, or oligonucleotide-conjugated dyed microparticles or colloidal gold. Detection of amplicon involves the "detection oligonucleotide" of the detection probe that is complementary to or otherwise able to bind specifically to the amplicon to be detected. Conjugation of a detection oligonucleotide to a microparticle can occur by use of streptavidin coated particles and biotinylated oligonucleotides, or by carbodiimide chemistry whereby carboxylated particles can be activated in the presence of carbodiimide and react specifically with primary amines present on the detection oligonucleotide. Conjugation of the detection oligonucleotide to the detectable moiety can occur internally or at the 5' end or the 3' end. Detection oligonucleotides can be attached directly to a detection label (e.g., the portion of the detection probe that provide for a detectable emission such as fluorescence, color, etc.), or more, for example, through a spacer moiety such as ethylene glycol or polynucleotides.

Once the amplicons are bound with the labeled probes, labeled amplicons are created and can be detected at 140. In an embodiment, the detection occurs through the a capturing moiety configured to bind to and selectively capture the labeled amplicons. Such capturing moiety can be provided on a solid or semi-solid phase that the labeled product is flowed into contact with, such as, but not limited to, for example, a lateral flow substrate, a gel, surfaces of a chamber or channel, a particulate bed, corrugated structures, etc. Depending on the labeling technique employed, detection of labeled amplicons can be via observation of color development (colorimetric detection) or sensing fluorescence by an optical sensor, or a combination of both.

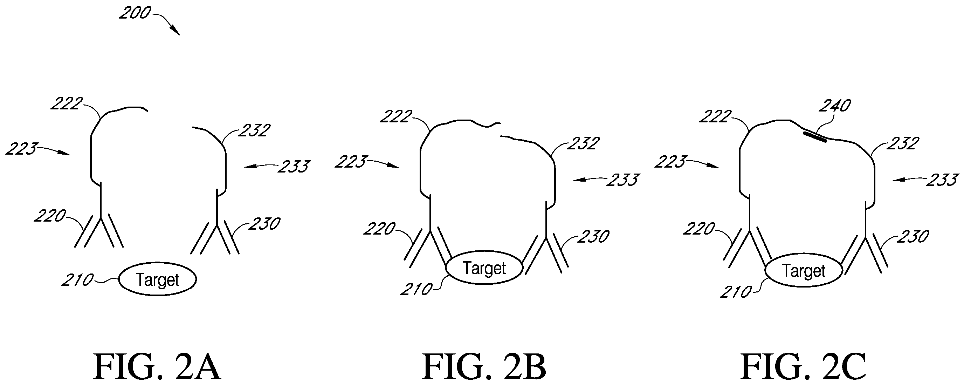

As described for step 110 of FIG. 1, target-linked template generation provides template nucleic acid, which is then amplified in a subsequent amplification step, for example, as described for step 120 of FIG. 1. FIG. 2A through FIG. 2C schematically illustrate target-linked template generation for PLA in accordance with embodiments of the present disclosure. FIG. 2A shows various stages and components of PLA reaction 200 for generating a single strand of template nucleic acid from a target analyte 210 from which a double-stranded template nucleic acid can be generated for subsequent amplification. The components of the PLA reaction 200 depicted include a first antibody or antibody fragment 220 specific to a first epitope of a target analyte protein 210 and a second antibody or antibody fragment 230 specific to a second epitope of the target analyte protein 210. An antibody or antibody fragment can be substituted with a DNA aptamer or other binding moiety with specificity for the respective target epitope. A first oligonucleotide 222 is bound at its 5'-end to first antibody 220 with its 3'-end free to form a first antibody conjugate. A second oligonucleotide 232 is attached at its 3'-end to second antibody 230 with its 5'-end free to form a second antibody conjugate. In FIG. 2B, first and second antibodies 220, 230 bind to target analyte 210 due to their affinity for the respective first and second epitopes of target analyte 210. In FIG. 2C, with the first and second antibodies 220, 230 bound to analyte 210, a splint oligonucleotide 240, along with a ligase reagent, is used to ligate the end portions of the first and second oligonucleotides 222, 232. The result is a proximity oligonucleotide product that comprises the joined first oligonucleotide 222, the second oligonucleotide 232, and the third, splint oligonucleotide 240. The resulting proximity oligonucleotide product thus provides a single strand of the template nucleic acid (the joined strands 222, 232) for generating a double-stranded template to be used in an amplification reaction.

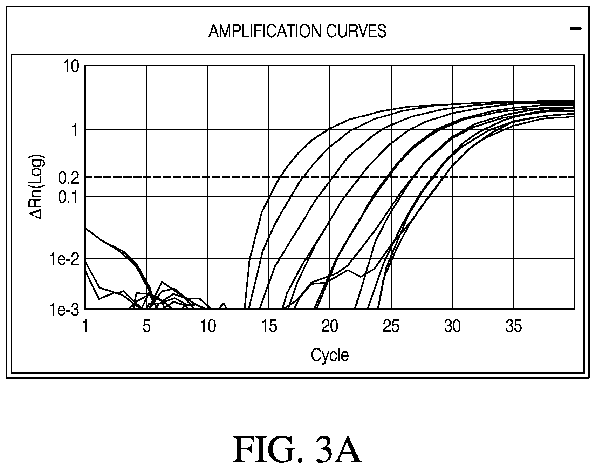

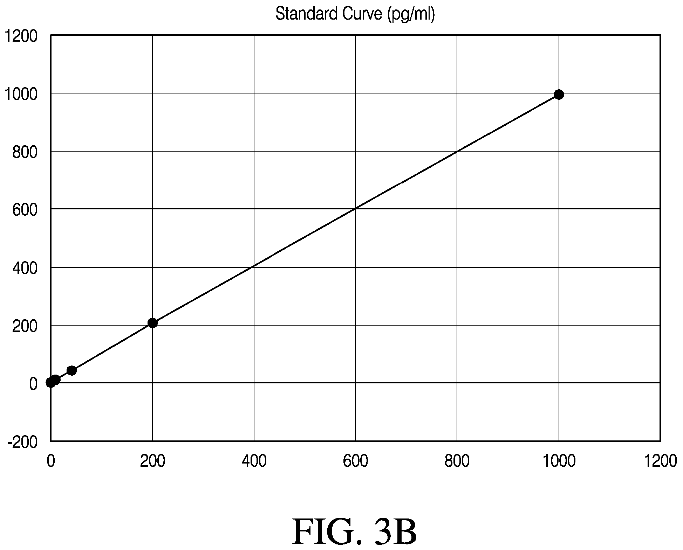

FIG. 3A is a graph including a series of illustrative curves of fluorescence v. cycle number in accordance with the present disclosure. This graph represents fluorescence v. cycle number curves for four hypothetical different starting protein concentrations, as reflected by the differing curves from left to right. The dashed line represents the fluorescence threshold. The cycle number required to reach the fluorescence threshold (threshold cycle or "Ct") can be plotted versus protein concentration to create a standard curve as shown in FIG. 3B. Thus, using PLA for target analyte-linked template nucleic acid generation and amplification in conventional qPCR format can achieve detectable amounts of labeled amplicons as a proxy for initial small concentrations of target analyte, providing the ability to increasing detectable emission to over, for example, a 1,000-fold. As will be described in more detail herein, the present inventors overcame challenges in adapting PLA to a lateral flow assay format to provide the detection level currently achievable with the qPCR format in a lateral flow assay format with visual detection. As such, the present disclosure provides for the advantages of detection limit achievable in the qPCR format in a point-of-care diagnostic platform with reduced sample handling, processing, and volumes needed to achieve such detection limits.

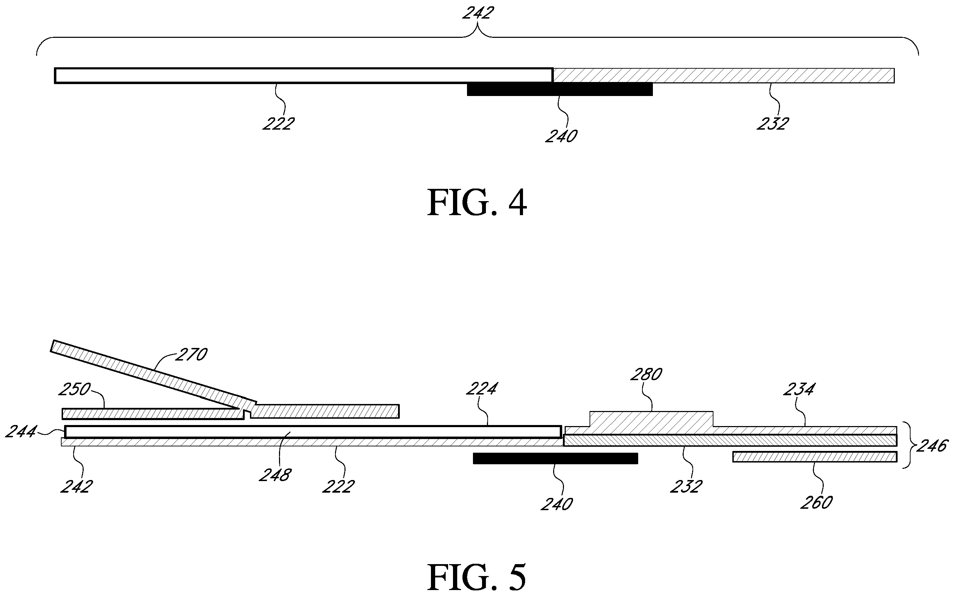

To aid in understanding of the proximity oligonucleotide strand that is produced, reference is made to FIG. 4 depicting a schematic diagram of the oligonucleotides and their relationships for the proximity ligation assay. FIG. 4 shows first oligonucleotide 222, second oligonucleotide 232, and splint oligonucleotide 240 in their joined form and from which the combined single strand 242 of template nucleic acid comprising the joined oligonucleotides 222, 232 is produced and can be used, as explained further below with reference to FIG. 5, to produce double-stranded template nucleic acid for amplification. In some embodiments, there is no split oligonucleotide, and the first oligonucleotide and the second nucleotide can be ligated to each other without and provide a single strand of the template nucleic acid. In some embodiments where a split oligonucleotide is present in the ligation step, the first and second oligonucleotide hybridize to the split oligonucleotide with no gap so that only a ligation would be needed to produce the template nucleic acid. In some other embodiments, the first and second oligonucleotides hybridize to the split oligonucleotide with one or more gaps of nucleic acids such that filling the gap would be needed prior to the ligation.

In some embodiments, the length of oligonucleotides that can be used herein can vary, e.g., from 2 bases to several hundred bases. For example, the length of first and second oligonucleotides such as 222 and 232 from the non-limiting, exemplary embodiment from FIG. 4, each of the oligonucleotides can be about 10 bases to about 1,000 bases. In some embodiments, the first and second oligonucleotides can be about 10 bases, about 25 bases, about 50 bases, about 75 bases, about 100 bases, about 125 bases, about 150 bases, about 175 bases, about 200 bases, about 500 bases, about 750 bases, about 1,000 bases or any intervening number of bases of the foregoing. In some embodiments, the length of forward and reverse primers such as 250 and 260 from FIG. 4 can be about 10 bases, about 25 bases, about 50 bases, about 75 bases, about 100 bases, about 125 bases, about 150 bases, about 175 bases, about 200 bases, about 500 bases, about 750 bases, about 1,000 bases or any intervening number of bases of the foregoing. In some embodiments, the length of primers can be about 35 bases to 75 bases, e.g., about 35 bases, about 40 bases, about 45 bases, about 50 bases, about 55 bases, about 60 bases, about 65 bases, about 70 bases, or about 75 bases. In some embodiments, the length of split oligonucleotide such as 240 from FIG. 4 can be about 2 bases, about 4 bases, about 10 bases, about 15 bases, about 20 bases, about 25 bases, about 30 bases, about 35 bases, about 40 bases, about 45 bases, about 50 bases, about 100 bases or any intervening number of bases of the foregoing.

Any or all stages of process 100 of FIG. 1 can utilize one or more reagent oligonucleotides. In addition to first oligonucleotide 222, second oligonucleotide 232, and splint oligonucleotide 240 shown in FIG. 4 that relate to the proximity ligation assay, oligonucleotides relating to the amplification, labeling, and detection are also shown. These comprise, for example, and with additional reference to FIG. 5, the generated first single template nucleic acid strand 242 from the joined first and second oligonucleotides 222, 232; second template strand 224 comprising the forward complementary to first oligonucleotide 222 and a third template strand 234 comprising the reverse complementary to second oligonucleotide 232, which form the single complementary strand 244 of template nucleic acid; the generated double-stranded template nucleic 246 comprising the hybridized first and second single strands of template nucleic acid 242, 244; first (forward) primer 250 and second (reverse) primer 260 used for amplification of double-stranded template nucleic acid 246 to generate an amplicon (amplification product); detection oligonucleotide of detection probe 270 that labels amplicon to generate a labeled product; and capture probe 280 that binds to the amplicon of template nucleic acid.

Once the ligase used in the PLA template generation reaction is inactivated, for example, by incubation at an elevated temperature in a range of about 95 °C, primers can be used to create a double-stranded template nucleic acid from the single strand of template nucleic acid and an amplification of the created double-stranded template nucleic acid can occur, such as, for example, via thermal cycling and PCR. In various embodiments, the amount of amplified nucleic acid product (amplicons of the template nucleic acid) can be measured after each amplification cycle via the use of fluorescent dyes and which exhibit fluorescence that is directly proportional to the number of PCR product molecules ("amplicons") generated.

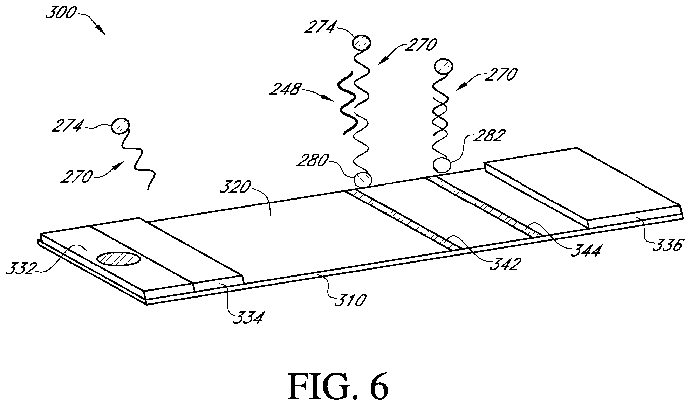

With the significant increase in amplified product achieved by analyte-linked template generation and amplification in view of process 100 of FIG. 1, such as via a proximity assay, labeling and detection of the amplified template nucleic acid product can occur and be used as a proxy for detection of relatively small amount of the target analyte of interest. In various embodiments, as noted above, because of the increased sensitivity, the amplicon labeling and detection of process 100 can be performed via lateral flow technologies. FIG. 6 is a schematic representation of one embodiment of a lateral flow labeling and detection system 300 that can be used for both the labeling and detection stages of an overall protein detection assay in accordance with an embodiment. In an embodiment, the lateral flow system 300 can be used with the resulting amplification product of the process 100, including but not limited to as carried out via a proximity ligation assay described above.

Lateral flow labeling and detection system 300 comprises a lateral flow substrate 310, which in the embodiment shown in in the form of a strip. A lateral flow substrate as used herein can refer to the overall components of a test strip with differing substrate layers to perform different functions, or to a porous matrix with the capture moieties for providing a readable emission (signal) for detection. Detection oligonucleotides can be affixed to microparticles, such as but not limited to colored bead 274, to form a detection probe 270, one being illustrated in the figure for simplicity to form detection particles. As previously described herein, colored bead 274 can be substituted with other types of colored beads or detectable microparticles, such as a visible or fluorescent dye attached to microparticles. Suitable detection probes include oligonucleotide conjugated to a detectable solid support, such as a polystyrene microspheres, latex beads or paramagnetic beads. Such detectable solid-supports can incorporate a range of detectable moieties including visible dyes, fluorescent dyes, quantum dots or colloidal gold.

In the embodiment shown, the lateral flow substrate 310 comprises a porous, absorbent matrix portion 320 that provides for capillary wicking of aqueous substances. The porous matrix 320 may be made of, for example but not limited to, cellulose, nitrocellulose, polyethersulfone, polyvinylidine fluoride, nylon, charge-modified nylon, or polytetrafluoroethylene. Attached to the porous matrix 320 are a surfactant pad 334 and an absorbent pad 336. These components can help establish capillary flow in the presence of a suitable liquid phase. The surfactant pad 334 can comprise amphipathic reagent to enhance wicking through the matrix 320, and the absorbent pad 336, attached at downstream end of the matrix 320 can further help to ensure flow of the aqueous solution of interest is completed through a length of the lateral flow substrate 310.

Conjugate pad 332 contains a reservoir of the detection probes 270 that are configured to bind to and label a target nucleic acid sequence 248, as will be explained in further detail below. Capture probes (moiety) 280 are contained in or on the porous matrix 320 and concentrated at a location defining a test line 342 extending transverse to the capillary flow direction through the lateral flow substrate 310. In other embodiments, the concentration of the capture probes can be in other shapes (e.g., dots, a plus sign, a minus sign etc.) or arrangements. Capture probes 280 are configured to capture the target nucleic acid sequence 248, which is bound to detection probe 270. When a sufficient level of target sequences 248 with their corresponding attached detection probes 270 are captured, the concentration of detection probes (e.g., colored beads 274) along test line 342 makes the test line 342 appear as the color of the colored beads 274 and visible to the eye of an observer. This development of color at the test line 342 thus provides a colorimetric indication of presence of a detectable concentration of the target sequence 248.

As shown in the embodiment of FIG. 6, the porous matrix 320 can further contain in or on it control probes 282 along a control line 344 (or concentrated in any desired arrangement as described above for test line 342). The control probes 282 have a binding affinity for the detection probes of 270 and thus capture the detection probes 270 in the absence of the target sequence 248. In other words, the test line 342 acts as a filter to first capture any detection probes 270 bound to target sequence 248 due to the capture probes 280 affinity for binding with the target sequence 248, but any detection probes 270 not bound a target sequence will not be captured at test line 342 and instead be captured by the control probes 282 concentrated at the location defining control line 344. By setting predetermined concentrations of capture probes 280 and control probes 282, color developed at either of test line 342 or control line 344 can indicate the presence of or presence in sufficient concentration of the target sequence 248. Capture probes for performing other capturing of control probes to indicate fault conditions of the lateral flow system for detection can also be used.

By providing detection probes 270 with a moiety (e.g., detection oligonucleotide) that has an affinity to bind with the amplicons of template nucleic acid generated, for example, via process 100 of FIG. 1, the detection probes 270 can thus label the amplicons of the template nucleic acid by placing the amplified product into contact with the conjugate pad 332 (the amplicons thus being the target sequence 248 depicted in the illustration of FIG. 4). Due to the lateral capillary flow through the lateral flow substrate 310, the labeled amplicons can flow through the porous matrix 320 and into contact with the capture probes 280 at test line 342. In the presence of the labeled amplicons, the capture probes 280 capture the same due to the affinity to bind to the capture probes 280 and color thereby develops at test line 342, as described above. On the other hand, if no or an insufficient level of amplicons were generated in the template nucleic acid generation and amplification stages of process 100, then sufficient concentration of detection probes 270 would not be captured by the capture probes 280 at test line 342. The flow of the free detection probes 270 (i.e., not bound to an amplicon of the template nucleic acid) would instead arrive at control line and be captured by the control probes 282 in sufficient concentration to develop color along the control line that is visible to the eye of an observer.

### LATERAL FLOW DEVICES AND SYSTEMS FOR PROTEIN ANALYSIS

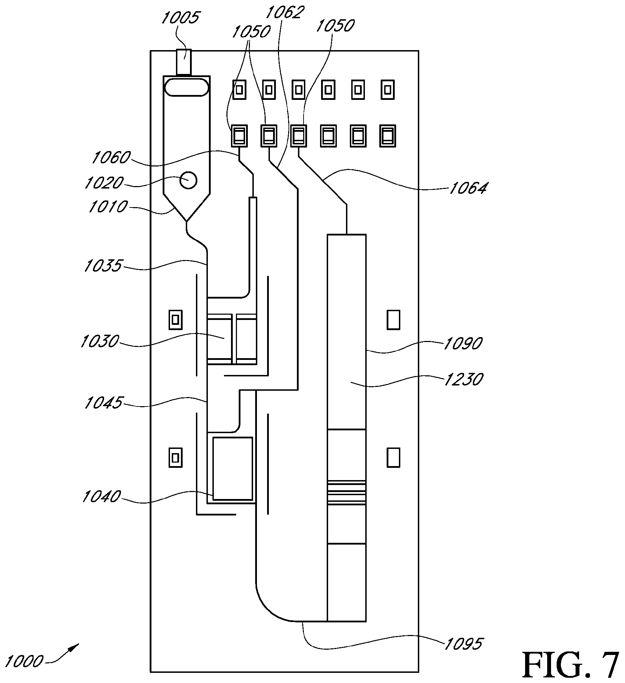

FIGS. 7-14 are directed to various embodiments that utilize a fluidic device for carrying out a protein detection assay, such as process 100 of FIG. 1, in accordance with the present disclosure, in which the fluidic device comprises a disposable fluidic cartridge that comprises a fluidic layer for sample flow, reagent introduction, and reactions and an electronic layer that can provide actuation of valving, temperature cycling and sensing, of the fluidic device. Overall process control algorithms and power can be provided through a docking unit, as described further below, that interacts with the electronics layer of the fluidic device.

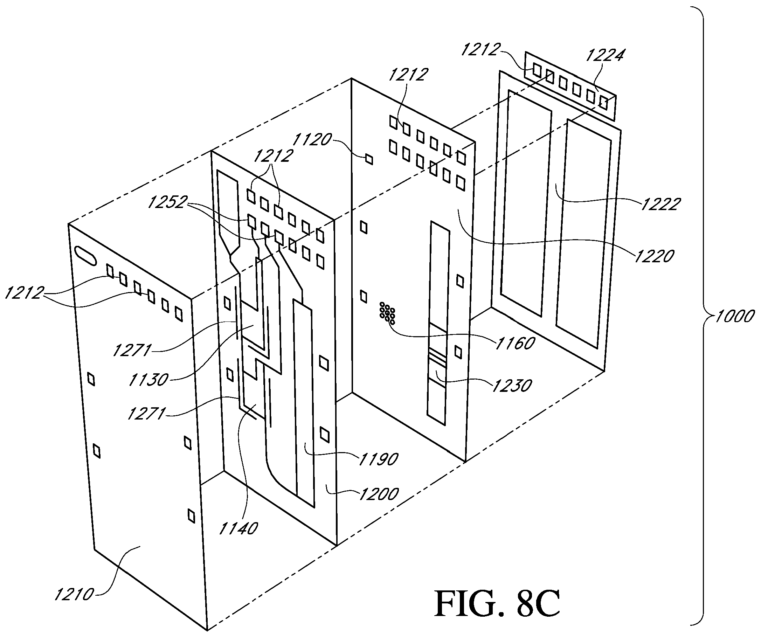

Referring to FIG. 7, fluidic component 1000 can include a fluidic layer, such as fluidic layer 1200 of FIG. 8C. Fluidic component 1000 comprises sample chamber 1010 to which sample can be introduced and initially collected in the fluidic component 1000 through inlet port 1005 of the device. The introduced sample can be added with or mixed with reagent already present in the sample chamber 1010, or alternatively reagent can be added to sample chamber 1010 after introduction of the sample. In various embodiments, the reagent can be in liquid or dry form and can contain components for target-linked template generation and/or template amplification, such as antibody conjugate reagents, buffering agents, salts, dNTPs, rNTPs, oligonucleotide primers, and/or enzymes. In other embodiments, the reagent can be or include a lysing reagent, which may be used in applications in which the sample introduced to sample chamber 1010 may need to be lysed, although it is also contemplated as within the scope of the present disclosure that lysing may occur as a preprocessing step prior to introduction of sample to the fluidic component 1000. The reagent mix can be lyophilized and be in the form of a pellet 1020. Introduction of the sample to the sample chamber can thus cause reagent and samples to effectively mix such that to provide a uniform solution and thereby a uniform reaction mixture. A bubble-mixing step to further mix the sample with the reagent or resuspend the reagent can be performed, as is described in WO 2012/145730. In other embodiments, the chamber 1010 may be free of any reagent mixing and simply used for a place to hold the sample prior to carrying out remaining reagent mixing and processing of the sample in other portions of the fluidic device.

Downstream of chamber 1010 and connected thereto via a conduit or channel 1035 is a first processing chamber 1030, and downstream of chamber 1030 is a second processing chamber 1040 connected by conduit or channel 1045. Downstream of second processing chamber 1040 is a detection chamber 1090 connected to second processing chamber 1040 by conduit or channel 1095. Each of the chambers 1030, 1040, and 1090 is connected by respective vent conduits or channels 1060, 1062, and 1064 to vent pockets 1050A, 1050B and 1050C, respectively. Vent pockets 1050A, 1050B and 1050 are able to place the vent channels 1060, 1062, 1064, and their corresponding connected chambers 1030, 1040, and 1090 into fluid communication with pressure external the fluidic component 1000 (e.g., ambient pressure) to cause transient pressure differentials in the chambers 1030, 1040, 1090 that combine with gravitational forces to promote fluid flow through the device, as is further explained below. According to the present disclosure, the terms "conduit" and "channel" and derivatives thereof, can be used interchangeably for fluidic elements providing, for example, but not limited by, fluid communication between various chambers, vent pockets, and the like.

As described with reference to various other embodiments, and further below, including with reference to FIG. 8C, detection chamber 1090 can house a lateral flow substrate, such as lateral flow substrate 1230. However, it is envisioned as within the scope of the present disclosure that detection chamber 1090 can be a chamber comprising a capture moiety provided on other than a lateral flow substrate and/or otherwise be configured to contain labeled amplicon for detection by an of the variety of techniques described herein, such as other colorimetric techniques and/or fluorescent detection techniques.

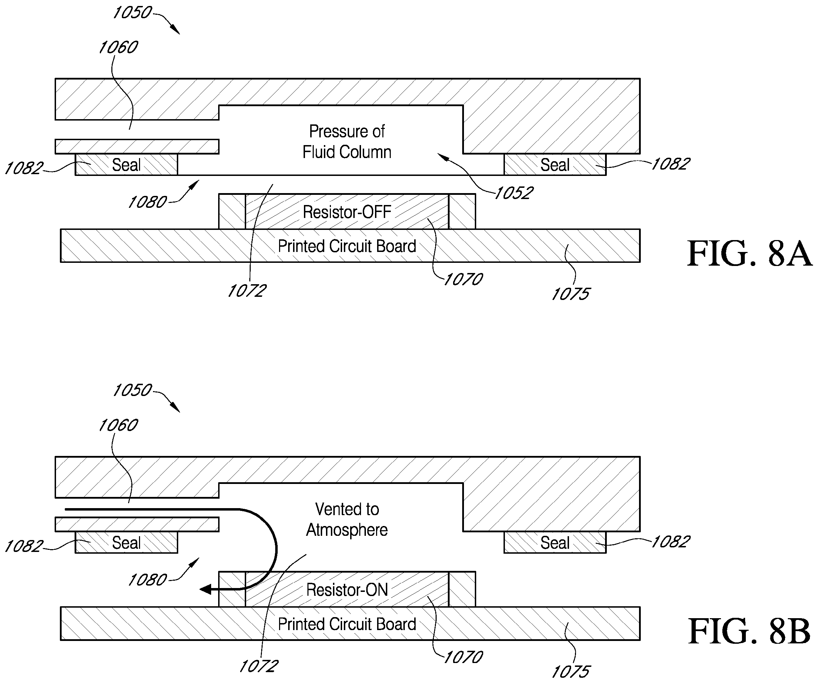

The various vents pockets, such as vent pockets 1050A, 1050B, and 1050C of the fluidic component 1000 are controlled via one-way valving mechanisms as will be explained further with reference to the partial cross-sectional views of FIGS. 8A and 8B, schematically depicting vent pockets. The term "vent" and the term "vent pocket" and derivatives thereof, are used interchangeably herein.

The vent pockets hold the pressure of the fluid column in the corresponding chamber coupled to the vent pocket 1050 above ambient. When actuated, the valve of the vent pocket 1050 opens and allows pressure in the chamber to equilibrate with ambient (i.e., the chamber pressure is vented to the exterior of the fluidic device). Fluid thus moves from an upstream chamber into its downstream connecting channel or conduit and into the chamber connected to the released vent pocket due to the hydrostatic pressure gradient that results and enables a displacement of higher pressure environment in the downstream connected chamber. Pressures can be less than a few mBar to achieve the fluid flow function.

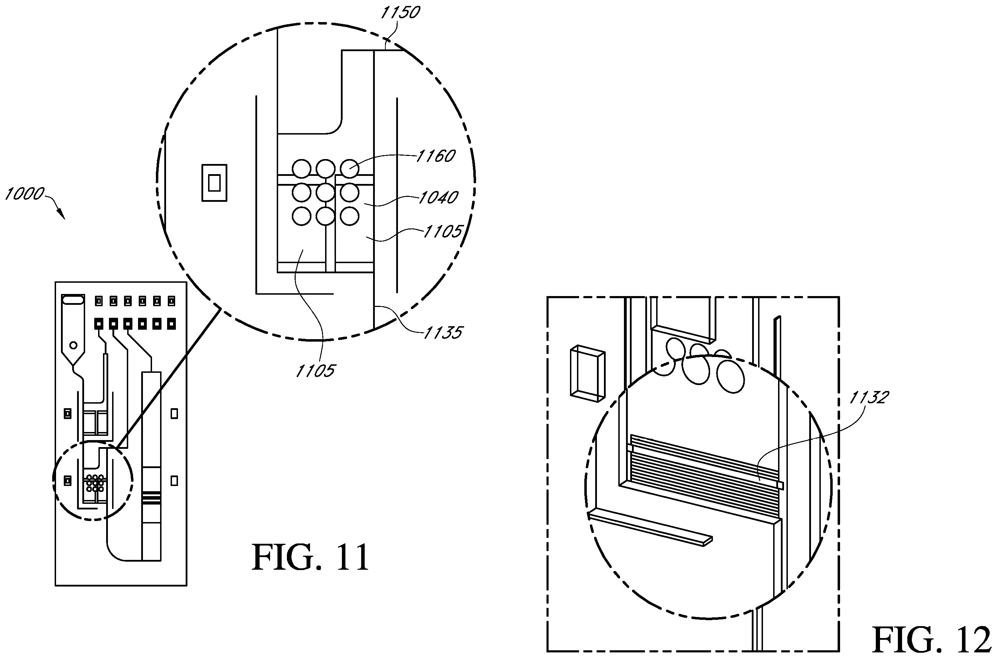

With further reference to FIG. 8A and FIG. 8B, generalized vent pocket 1050 is illustrated. Vent pocket 1050 is in fluid communication with a vent channel in fluid communication with a chamber of fluidic device, for example vent channels 1060, 1062, and 1064 of FIG. 7. Vent pocket 1050 can be formed as a recessed chamber in of a fluidic device and be open at a side facing the electronic layer 1075. The open side of vent pocket 1050 is initially covered with heat-labile membrane 1080. Heat-labile membrane 1080 may be sealed to the fluidics layer on either side of the pocket 1050 via seals 1082, which may be via adhesive or other forms of bonding. Aligned with the heat-labile membrane 1080, the electronic layer 1075 of the fluidic device, which may be printed circuit board assembly (PCA), can include a resistive heater 1070 mounted to the printed circuit board assembly (PCA). A microcontroller of the PCA, which may be operated and controlled via coupling through a docking unit as described with reference to the embodiment of FIGS. 11 and 12 and embodiments further below, can be responsible for sending electrical current to the resistive heater 1070. Heat produced by the energized resistive heater 1070 melts or otherwise disrupts the membrane 1080, thus opening the vent pocket 1050 to ambient pressure, as depicted in FIG. 8B. Once open, a vent pocket creates a pressure differential in the fluidic layer of the fluidic device that allows fluid to flow from an upstream chamber to a downstream chamber connected to the vent pocket. For example, as depicted in FIG. 7 through FIG. 8B, chamber 1030 is connected to chamber 1010 through channel 1035, and chamber 1030 is connected to vent pocket 1050A through channel 1060. Further, as depicted in FIG. 7, chamber 1040 is connected to chamber 1030 through channel 1045, and chamber 1040 is connected to vent pocket 1050B through channel 1062. Finally, as depicted in FIG. 7, detection chamber 1090 is connected to chamber 1040 through channel 1095, and chamber 1090 is connected to vent pocket 1050C through channel 1064. Actuating the opening of a vent pocket provides a path for gas to escape from a downstream chamber and to equalize with ambient pressure, thereby permitting fluid to enter the downstream chamber.

In various embodiments, the present disclosure contemplates that a vent pocket, such as vent pocket 1050 of FIG. 8A and 8B, can be initially sealed by a mechanism other than the heat-labile membrane 1080, and can utilize other methods of breaking the seals, such as puncturing, tearing, or dissolving the same using various mechanisms that may be actuated through a PCA. By programming through use of a docking unit comprising memory and a processor programmed with instructions for supplying current selectively to various resistors (or other vent-valve actuation mechanisms), the vent pockets 1050 can be actuated as desired to control flow through the fluidic component 1000.

FIG. 8C depicts an exploded view of fluidic component 1000 of FIG. 7. As depicted in FIG. 8C, fluidic component 1000 can be an assembly of three laminated plastic sheets. Fluidic layer 1200 can include openings 1130, 1140, and 1190 that define chambers 1030, 1040, and 1090 as depicted in FIG. 7. Face component 1210 and backing component 1220 are laminated to fluidic layer 1200 to form fluidic component 1000. Face component 1210 can include holes 1212 for viewing LED indicators, which can be powered via an electronic layer. Backing component 1220 can comprise one or more of lyophilized reagents 1120, detection probes 1160, and lateral flow substrate or other capture device 1230, and can interface with the electronics layer via an adhesive shim 1222, which can include membrane with adhesive border 1224. Acrylic- or silicon-based adhesives can be used in various embodiments. In general, adhesives can be used that are chemically compatible with various buffers, plastics and reaction reagents of the present disclosure, as well as being compatible and maintaining adhesive integrity during conditions and temperatures encountered during device operation.

In various embodiments, the thickness of the fluidic chambers and conduit or channel walls can be, for example, in the range of approximately 0.025 mm to approximately 1 mm, and in the range of approximately 0.1 mm to approximately 0.5 mm. The internal volume of chambers can range from, for example, between approximately 1 µl to approximately 50 µl. This thickness can provide both structural integrity of the fluidic layer and to support sealing of the closed chambers and channels under elevated temperatures and associated pressures. The thickness of channel walls, particularly vent channel walls, can be less than that of the chambers and in the range, for example, of approximately 0.025 mm to approximately 0.25 mm. The width of channels connecting chambers to one another can be chosen to enhance capillarity. A shallow vent channel can impart rigidity to the fluidic layer minimizing adverse effects on venting to atmospheric pressure. Plastic forming faces of the fluidic layer can be thinner than that forming the walls in order to maximize heat transfer. Thermal breaks 1271 can cut through some components of the fluidic layer and surround the amplification and detection chambers, contributing to the thermal isolation of the temperature-controlled chambers.

Vent pockets, such as vent pockets 1050 described with reference to FIG. 8A, can be differentiated from other reaction chambers in their construction. After construction of the fluidic layer as described above, vent pockets possess an open face on the side of the fluidic layer that will meet with the electronics layer. To form the vent pocket, fluidic layer 1200 comprises openings 1252 smaller than but similar in construction to the openings that form chambers. Those openings 1252 align with openings 1152 provided in the backing component 1220. An additional plastic component is laminated to seal the chamber, and comprises the heat-labile membrane (e.g., membrane 1080 in FIGS. 8A-8B) with one adhesive face for application to the fluidic layer side adjacent to vent resistive heating element of the electronics layer. In an embodiment, the vent heat-labile membrane can comprise polyolefin of ranging from approximately 5 picometers (pm) to approximately 200 pm thickness, although other similar films can be used. The heat-labile membranes should be selected so as to both provide robust sealing of the vent pockets under operating conditions of the fluidic device, while allowing for easy perforation and, thus, venting to the atmosphere when current is passed through the vent resistor generating a rapid temperature increase.

Components of fluidic component 1000 can comprise plastics, for example, such as acrylic, polycarbonate, PETG, polystyrene, polyester, polypropylene, and/or other like materials. Plastics used in the assembly of the fluidic layer, such as acrylic and polyester, can comprise hydrophobic materials. Components of the fluidic layer can be treated to enhance wettability (i.e. decrease hydrophobicity). Such treatments can facilitate proper fluid control in conjunction with fluidic channel dimensions. A biocompatible surfactant such Triton X-100 can be applied to uncoated materials. Plasma etching can be alternatively used to alter the hydrophobicity of fluid contacting surfaces.

As described above, several additional components can be incorporated within fluidic layers of fluidic device in accordance with embodiments of the present disclosure before final lamination and sealing. Reagents including buffers, salts, dNTPs, oligonucleotide primers, and enzymes such as DNA polymerase and reverse transcriptase can be lyophilized, or freeze-dried, into pellets or cakes prior to device assembly. Reagent lyophilization involves dehydration of frozen reagent aliquots by sublimation under an applied vacuum. By adding specific formulations of lyoprotectants such as sugars (di- and polysaccharides) and polyalcohols to the reagents prior to freezing, the activity of enzymes can be preserved and the rate of rehydration can be increased. Lyophilized reagent pellets or cakes can be manufactured by standard methods, and once formed, can be reasonably durable and can be placed into or proximal to specific chambers of the fluidic layer prior to laminating the final face.

Detection probes can be another additional component of the fluidic layer. For example, microparticles can be lyophilized as described for the labeling reaction reagents above. Microparticles in liquid buffer can be directly applied to an interior face of a fluidic chamber and dried before sealing. The liquid buffer containing the detection probes can also comprise sugars or polyalcohols that aid in rehydration. Incorporation of detection probes in aqueous buffer directly into the fluidic layer prior to drying can be employed. Heating or nucleate boiling (bubble formation) as described above can be used to both adequately mix the detection particles with the reaction solution, and to denature double-stranded nucleic acid product for hybridization to the detection probes.

A lateral flow substrate 1230 for detection can also be incorporated into backing component 1220 of the fluidic layer. As described above, in various embodiments the lateral flow substrate can comprise a membrane assembly comprised of at least one porous component with a detection zone, and can include one or more of an absorbent pad, a surfactant pad, a backing film (e.g., a plastic or other film to prevent moisture from wicking through the substrate toward the electronics layer), and a conjugate pad (the latter being used for when labeling occurs at the detection chamber). Any of the lateral flow substrates described with reference to embodiments above may be used.

In various embodiments, a surfactant pad of a lateral flow substrate can comprise a porous material, tending to minimize nucleic acid binding and fluid retention properties so as to facilitate unobstructed migration of the labeled nucleic acid product (including detection probes) to the porous matrix comprising the detection zone. The surfactant pad can comprise materials such as glass fiber, cellulose, or polyester. Formulations including at least one amphipathic reagent can be dried on the surfactant pad to allow uniform migration of sample through the detection zone. The absorbent pad can comprise any absorbent material and helps to induce sample wicking through the lateral flow substrate.

In addition to the resistive heating elements for the vent pockets and the current thereto, the electronic layer of the fluidic component 1000 can include additional electrically-based heater or cooler elements in contact with various chambers of the fluidic layer of the fluidic component 1000 to provide a means for the temperature regulation. For example, a heater element may be aligned with a chamber to support nucleic acid amplification and may be configured to be controlled by the PCA and docking unit to cycle the heater element through various temperature profiles as needed to support a PCR reaction.

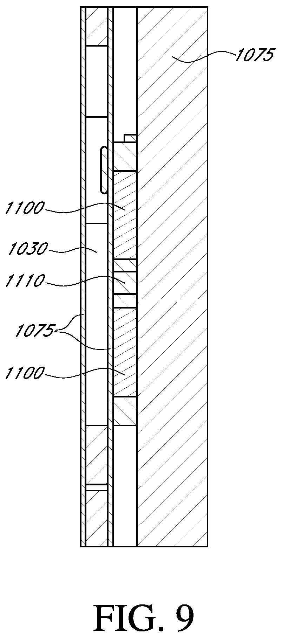

FIG. 9 shows further details of a resistive heater element useful for temperature regulation of a processing chamber, such as a chamber (e.g., chamber 1030) for performing amplification of fluidic component 1000 or any chamber in which heating above ambient may be desired. FIG. 9 is a partial, cross-sectional view showing the fluidics layer and electronics layer of a fluidic device such as fluidic component 1000. One or more film surface mount device (SMD) resistive heating elements 1100 can be aligned with portions of a chamber such as the amplification chamber 1030 of fluidic component 1000, with two such devices 1100 being shown in FIG. 9. A thermistor (temperature sensor) 1110 can also be included on the PCA and aligned with the chamber 1030. In the embodiment shown, the temperature sensor 1110 can be flanked by the two heating elements 1100, though other arrangements are also within the scope of the disclosure.

Various embodiments of the present disclosure can thus comprise heating and/or cooling elements, and corresponding temperature sensors, on a disposable fluidic device of an overall system, below. Placing the heating and/or cooling elements and corresponding temperature sensor(s) on a disposable component enables the manufacture of highly reproducible thermal coupling between the temperature control subsystem and the amplification and detection chambers to which they interface. This approach enables a relatively reliable means of coupling the fluidic subsystem to the electronic thermal control subsystem by forming the thermally conductive interface during manufacture. The resulting thermal contact between the electronic temperature control components and the fluidic subsystem can result in rapid temperature equilibration, and therefore improve the overall time taken to perform a protein detection assay, which is a factor in consideration of point-of care applications and other single-use applications.

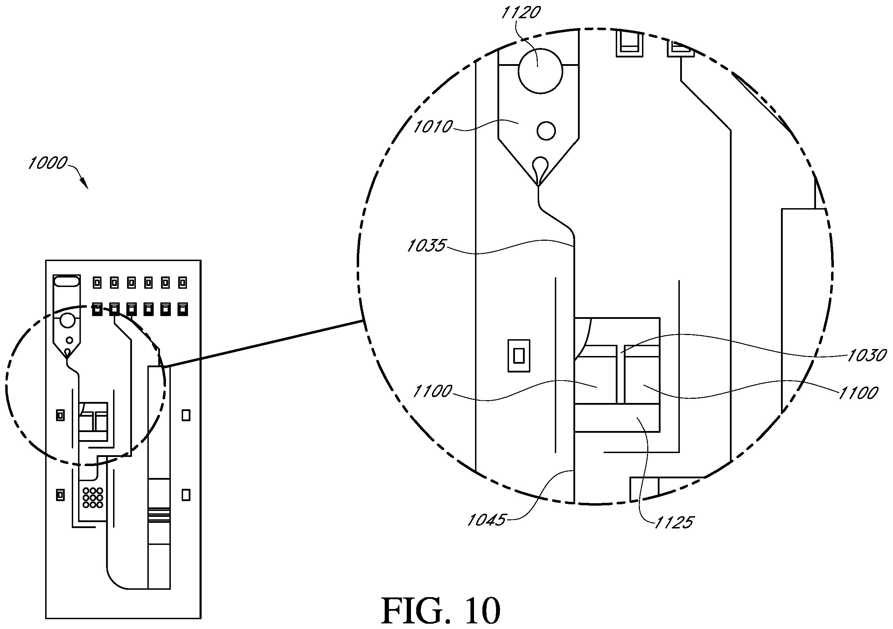

Resistive heating elements associated with chambers of a fluidic device, such as fluidic component 1000, can also be used for fluid flow through the device. FIG. 10 illustrates an embodiment of the present disclosure in which transport of fluid occurs using a mechanism other than the valve-vents described above. Using chamber 1030 of fluidic component 1000 to explain the principle or operation, the resistive heating element(s) 1100 (shown through the transparent plan view of FIG. 10) can be used to heat the chamber 1030. Such heating will cause gas contained in chamber 1030 to expand and purge itself through the conduit or channel 1035 connecting chambers 1010 and 1030. That is, the expanding gas will rise through the channel 1035 in the orientation of the fluidic component 1000 in use for performing the detection assay and enter the upstream chamber 1010 as gas bubbles 1120 within the fluid contents contained in the chamber 1010. By turning the resistive heating element(s) 1100 off, the remaining gas in the chamber 1030 volume will cool and contract, thereby drawing in a volume of fluid contents from the chamber 1010 through channel 1035 and into chamber 1030 proportional to that of the purged gas. Again, due to the orientation of the fluidic component 1000 in use, the volume of the contents 1125 from chamber 1010 drawn into chamber 1030 collects at the floor of chamber 1030 above the channel 1045. Successive iterations of temperature cycling as described above can move the full sample contents volume of chamber 1010 to chamber 1030. The operation of this technique depends on conduit or channel size, length, heat time and temperature, chamber volumes, and surface energies of components. This technique can also be used in conjunction with the vent-valve technique for causing fluid flow previously described herein.

In addition to driving flow of contents from one chamber to the next, the bubble formation caused by resistive heating and expansion of gasses can be used for promoting mixing of the contents present in a chamber. Thus, the technique can be used to promote enhanced mixing due to motion and displacement of contents in the chamber in which the bubbles are formed, which may assist in better mixing of sample and reagent in a chamber.

In various embodiments a microcontroller of the electronic layer (PCA 1075 of FIG. 8B) modulates current to the resistive heating element(s) for example by means of metal oxide semiconductor field effect transistors (MOSFETs), based upon data collected from temperature sensor 1110, for example by using on/off or proportional integral derivative (PID) temperature control methods or other algorithmic temperature control. A thin layer of thermal compound at the interface of the resistor(s) and the amplification chamber 1030 (or any chamber associated with such a heating element) can be used to enhance thermal conductivity to the heaters and sensor. Dimensions of the chamber can be chosen to maximize the area/volume ratio to achieve more uniform and rapid heating and cooling.

The overall structure, including number and arrangements of the chambers in the fluidic component 1000 can vary according to the various processes to be conducted in the device to carry out a protein detection assay, such as process 100 of FIG. 1. By way of non-limiting example, using the arrangement of FIG. 7, chamber 1010 may be used for sample introduction and template nucleic acid generation, chamber 1030 for amplification, chamber 1040 for labeling (described further below with reference to FIG. 11), and chamber 1090 for detection (e.g., with a lateral flow substrate). Alternatively, in some embodiments, chamber 1010 can be for sample introduction only and any preprocessing conditions that may be needed, chamber 1030 may be used for template generation, chamber 1040 for amplification, and chamber 1090 for labeling and detection, such as by utilizing a lateral flow substrate that includes a conjugate labeling pad as described with reference to the embodiment of FIG. 6. In yet other embodiments, chamber 1010 can be for sample introduction and any preprocessing conditions that may be needed, chamber 1030 can be used for both template nucleic acid generation and amplification, chamber 1040 for labeling, and chamber 1090 for detection.

In still another non-limiting example, using the arrangement of FIG. 7, sample chamber 1010 can be for sample introduction and binding with antibody reagents, where only one of chambers 1030 and 1040, can be used for template generation and amplification, with chamber 1090 being used for labeling and detection. In such an exemplary device, for example, where chamber 1030 is used for template generation and amplification, chamber 1040 can be used for postprocessing a sample formed from template generation and amplification in chamber 1030. As will be described in more detail herein, the present inventors have implemented PLA into a lateral flow format by overcoming a challenge of precipitation occurring in the chamber in which template generation and amplification were performed. Accordingly, postprocessing of a sample can include, for example, a treatment of the sample in a third chamber with a protease reagent, a chaotropic reagent, a surfactant reagent, an ampholyte reafent, or combinations thereof. Additional descriptions for forming some of the arrangements and chambers for the various uses described above are set forth in more detail below.

Embodiments of a chamber in which amplification is to be conducted can comprise materials capable of withstanding repeated heating and cooling to temperatures in the range of from approximately 30°C to approximately 110°C, and on the order of from approximately 10°C to approximately 50°C per second. An amplification chamber can be capable of maintaining solutions therein at temperatures suitable for either thermal cycling and/or isothermal amplification protocols, depending on the desired application of the fluidic device. In some nucleic acid amplification applications, it can be desirable to provide an initial incubation at an elevated temperature, for example in a temperature range from approximately 37 °C to approximately 110°C for a period of from approximately 1 second to approximately 5 minutes, to denature the template nucleic acid. Subsequently, the reaction solution can be varied in temperature between at least two temperatures including, but not limited to, a temperature that results in nucleic acid duplex denaturation and a temperature suitable to primer annealing by hybridization to the target and extension of the primer through polymerase catalyzed nucleic acid polymerization. The duration of incubations at each requisite temperature in a thermal cycling regimen can vary with the sequence composition of the template nucleic acid and the composition of the reaction mix, and can, for example, be range from approximately 0.1 seconds to approximately 20 seconds. Repeated heating and cooling can be typically performed for approximately 20 cycles to approximately 50 cycles. In embodiments involving isothermal amplification methods, the temperature of the reaction solution can be maintained at a constant temperature (in some cases following an initial incubation at an elevated temperature) ranging from approximately 3 minutes to approximately 90 minutes depending on the amplification technique used. Once the amplification reaction can be complete, the amplification reaction solution can be transported, for example by the vent-valve and/or gas bubble techniques described above with reference to FIG. 10.

Additional biochemical reactions can be conducted in the amplification chamber prior to, during, or after the amplification reaction. Such processes can include but are not limited to a target-linked template nucleic acid generation (e.g., including via PLA or PEA), reverse transcription wherein RNA can be transcribed into cDNA, multiplexing wherein multiple primer pairs simultaneously generate and amplify multiple template nucleic acids, and real time amplification wherein amplification products can be detected during the amplification reaction process. The amplification chamber can be aligned with a window or otherwise be configured to enable interrogation of amplicon concentration during the amplification reaction process. Either fluorescently labeled oligonucleotides complementary to the template nucleic acid or fluorescent dyes specific for duplex DNA can be monitored for fluorescence intensity by means of an excitation light source such as LEDs or diode laser(s) and a detector such as a photodiode, and appropriate optical components including but not limited to optical filters. In various embodiments, such components may be part of an external system, such as a docking unit.

Detection probes can provide for the specific labeling of amplified template nucleic acid generated through preceding target-linked template nucleic acid generation and amplification, in which labeling can occur upstream of a detection chamber, such as detection chamber 1090 of FIG. 7. As shown in FIG. 11, fluidic component 1000 can include detection probes 1160, shown here in 1040 (see also FIG. 7). Detection probes 1160 that can be dried, lyophilized, or present on at least a portion of the interior surface as a dried mixture of detection probes in a carrier such as a polysaccharide, detergent, protein, or other compound to facilitate resuspension of the detection probes. Chamber 1040 can be connected to inlet channel 1135 leading to detection chamber 1090 (see FIG. 7). As with other chambers to which it is desired to flow contents from an upstream chamber, in various embodiments, the chamber 1040 is connected to a vent pocket through a vent channel 1150. One or more resistive heating elements 1105 and corresponding temperature sensor (thermistor) from the PCA layer may be aligned with the chamber 1040. Further, in various embodiments, at the interface with the PCA, a thin layer of thermally conductive material such as thermal grease may be disposed between one face of the chamber 1040 and a resistive heating element.

In the case of a double-stranded DNA amplification product, heating the reaction solution following introduction to the detection chamber facilitates detection. Melting the double-stranded DNA and then cooling in the presence of detection oligonucleotide of the detection probes results in the sequence-specific labeling of the amplified template nucleic acid. The resistive heating element(s) aligned with the chamber 1040 can be used to heat the fluid volume for approximately 1 to approximately 120 seconds to initiate double-stranded DNA melting. As the solution is allowed to cool to room temperature, the amplicons of the template nucleic acid can specifically hybridize to the detection probes. The reaction volume can then be directed to a region of the detection chamber located downstream of chamber 1040, e.g., by actuating the vent pocket associated with the detection chamber.

For efficient labeling, the solubilized detection probes 1160 can be mixed with the reaction solution by using resistive heaters associated with the labeling chamber. The resistive heaters can be actuated during labeling to both denature double-stranded nucleic acid target and sufficiently mix detection probes in the reaction solution. Heating of the solution during labeling to above the boiling point can be used to induce turbulence and mixing in solution. Rising bubbles nucleated at the bottom and sides of the chamber by a textured feature such as laser etched line 1132, shown in FIG. 12 (or a series of such lines), can effectively stir the solution. Solution displaced into upper, empty chambers by boiling can flow downstream back into chamber 1040 during subsequent cooling. Regions of the inner face or the chamber walls can be textured or otherwise treated to localize nucleate boiling to a specific chamber wall or face. One or more boiling chips can be placed in the chamber to localize nucleate boiling to a specific point(s).

As described above, a detection chamber of a fluidic device in accordance with various embodiments provides for the specific detection of labeled amplicon of the generated template nucleic acid. In the fluidic component 1000 in the embodiment of FIG. 7, detection is accomplished by use of a lateral flow substrate, which involves capillary wicking of solution (i.e., in this case the solution containing the labeled amplicon) through an absorbent matrix comprised of a porous material (such as, but not limited to, for example cellulose, nitrocellulose, polyethersulfone, polyvinylidine fluoride, nylon, charge-modified nylon, or polytetrafluoroethylene). As described above, with reference to the embodiment of FIG. 6, the porous matrix can be patterned with lines, dots or other visually discernable shapes by concentrating capture probes capable of specifically binding to the labeled amplicon either directly or indirectly. As described with reference to FIG. 6, in various embodiments, the lateral flow substrate component in the detection chamber can comprise multiple porous substrates in physical contact, such as, a surfactant pad comprising amphipathic reagents to enhance wicking at an upstream portion, a detection zone comprising a porous material (such as cellulose, nitrocellulose, polyethersulfone, polyvinylidine fluoride, nylon, charge-modified nylon, or polytetrafluoroethylene) to which at least one capture moiety capable of selectively binding labeled amplicon can be immobilized (and additionally a capture moiety for binding detection probes not bound to amplicon), and/or an absorbent pad located at a downstream end of the lateral flow substrate to provide additional absorbent capacity to help ensure flow along an entire length of the detection zone.

A capture probe, including those used as control probes, can be immobilized to the porous matrix of the lateral flow substrate by any of a variety of means, for example, such as UV irradiation. The capture probe can be designed to capture the labeled template nucleic acid as solution containing the labeled template nucleic acid wicks through the detection zone resulting in an increased concentration of detection probes at the site of capture probe immobilization, thus producing a detectable signal indicative of the presence of the labeled amplicons of template nucleic acid. A single detection lateral flow substrate can be patterned with one or multiple capture probes to enable multiplexed detection of multiple amplicons, determination of amplicon sequence, and assay quality control (positive and negative controls).

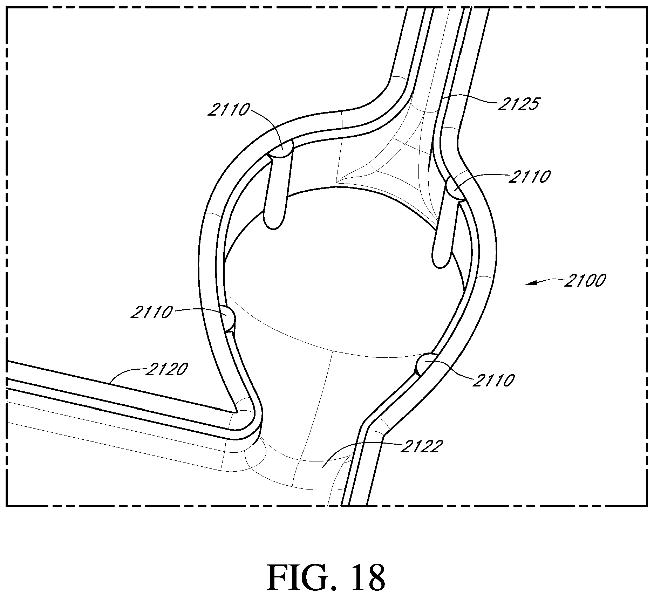

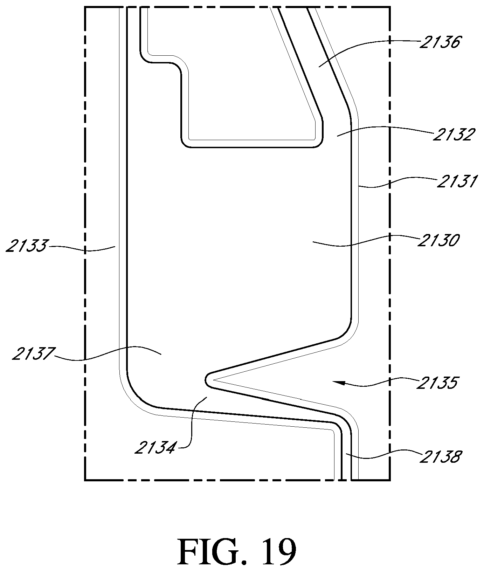

As will be appreciated from the description of various embodiments above, the lateral flow substrate of the detection chamber 1090 can be for detection only in the case in which one of the upstream chambers of the fluidic device (e.g., 1030 or 1040 in fluidic component 1000) is used as a labeling chamber, for example as described with reference to the embodiment of FIGS. 18 and 19. Alternatively, in embodiments in which the upstream chambers (e.g., 1030, 1040) are not used for labeling, the detection chamber 1090 can contain a lateral flow substrate that includes a conjugate pad and suitable labeling probes, such as, for example, the lateral flow test strip of the embodiment of FIG. 6.

Moreover, depending on the nature of the detection probes uses for labeling, the detection of the capturing of such probes in the detection chamber can be via colorimetric development and observation, or via fluorescence detection in the case of labeling using fluorophores. In yet other embodiments, the detection chamber may have mechanisms other than a lateral flow substrate for retaining capture probes therein to provide for suitable detection of whether or not labeled amplicon of template nucleic acid is captured at concentrations sufficient to determine the presence of the target protein in the initial sample.

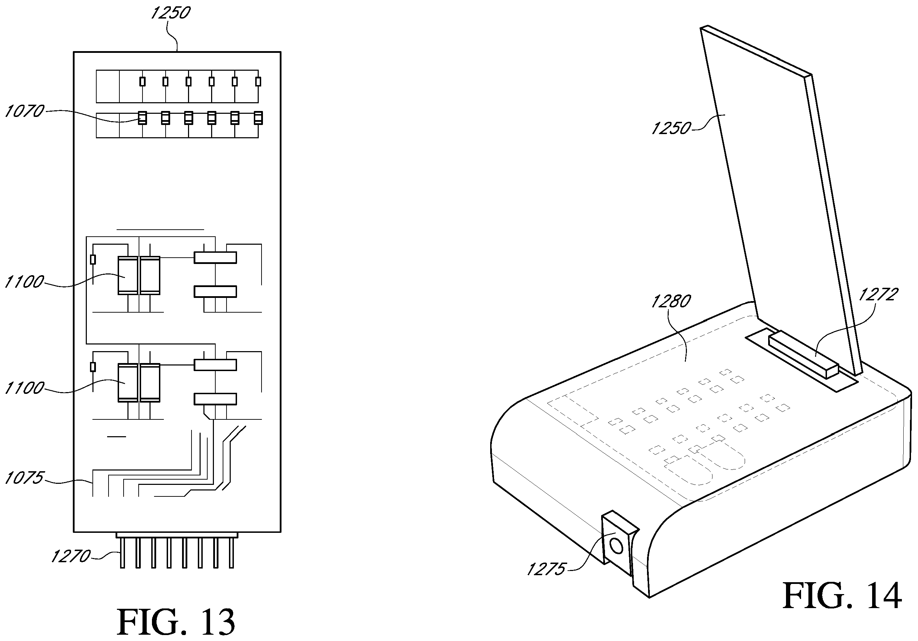

FIG. 13 illustrates fully-assembled fluidic device 1250, which includes a fluidic component, such as fluidic component 1000 of FIG. 7 and FIG. 8C. As depicted in FIG. 13, fluidic device 1250 can include electronic layer 1075, resistive heaters 1070, as previously described herein for FIG. 8C, as well as chamber heating elements 1100, as previously described herein for FIG. 9. Electronic layer 1075 of fluidic device 1250 can be integrated with a multi-pin connector, such as multipin connector 1270 of FIG. 13, or other suitable connector that can be electronically and communicatively coupled with a docking unit, such as docking unit 1280 of FIG. 14.

FIG. 14 illustrates fluidic device 1250 of FIG. 13 in place in docking unit 1280. Docking unit 1280 can provide the various control and power functionality for fluidic device 1250, as well as orienting fluidic device 1250 in a desired orientation. Various docking units of the present disclosure are portable and handheld. As depicted in FIG. 14, fluidic device 1250 can be inserted in connector 1272, for example, in a vertical orientation. Docking unit 1280 can include a user interface including an LED, or LCD display, as well as features offering user control. For example, docking unit 1280 can include buttons to initiate electronic processes used for the assay.

As described above, the electronic functions can thus be split into two separate subassemblies. The disposable fluidic device subassembly comprising the fluidic and electronic layer 1075 and the reusable docking unit 1280, which can incorporate reusable components such as the microcontroller, MOSFETs, switches, power supply or a power jack 1275 and/or battery, cooling fan, user interface, and connector 1272 compatible with connector 1270 of the fluidic device. When the subassemblies are mated via connectors 1270 and 1272, docking unit 1280 can support the fluidic device in a substantially vertical or near-vertical orientation. Although a substantially vertical orientation can be used, similar results can be obtained if the device is operated at a tilt, especially if certain pathways can be coated to reduce the wetting angle of solutions used. Examples of a tilt include about 1° from vertical, about 2.5° from vertical, about 5° from vertical, about 7.5° from vertical, about 10° from vertical, about 15° from vertical, about 20° from vertical, or about 30° from vertical, or any intervening range or value.

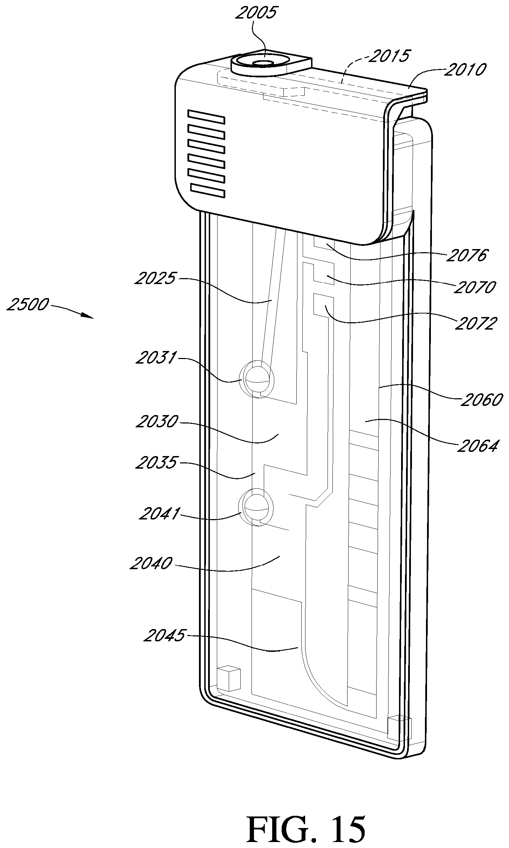

FIG. 15 is a perspective view of an embodiment of a fluidic device that can be utilized for performing a protein detection assay of the present disclosure. Fluidic device 2000 includes sample port 2005 providing entry of a sample into fluidic device 2500. Cover 2010 is configured to hold sliding seal 2015 in place in order to seal fluidic device 2500 during use. As will be described in more detail herein, sliding seal 2015, shown in phantom view in FIG. 15, is moved to a closed position at the time of assay initiation.

Fluidic device 2500 as depicted in FIG. 15, during use, when fluidic device 2500 is oriented in a substantially vertical orientation, a sample introduced into sample port 2005 is directed through first fluidic channel 2025 and through second reagent recess 2031 (first reagent pellet not shown in FIG. 15) into first processing chamber 2030. Second fluidic channel 2035 of FIG. 15 connects first processing chamber 2030 to third reagent recess 2041. Following sample processing in chamber 2030, vent 2072 is opened to allow the sample to flow via second fluidic channel 2035 into third reagent recess 2041. As will be described in more detail herein, second reagent recess 2031 and third reagent recess 2041 may contain reagents used for a first processing and a second step in the respective processing chambers. Reagents can be dried or lyophilized to form a reagent pellet in each reagent recess. Following sample processing in second processing chamber 2040, vent pocket 2076 is opened to allow the sample to flow via third fluidic channel 2045 into detection chamber 2060. As will be described in more detail herein, lateral flow strip 2064 situated in detection chamber 2060 enables the detection of target nucleic acids labeled by detection particles.

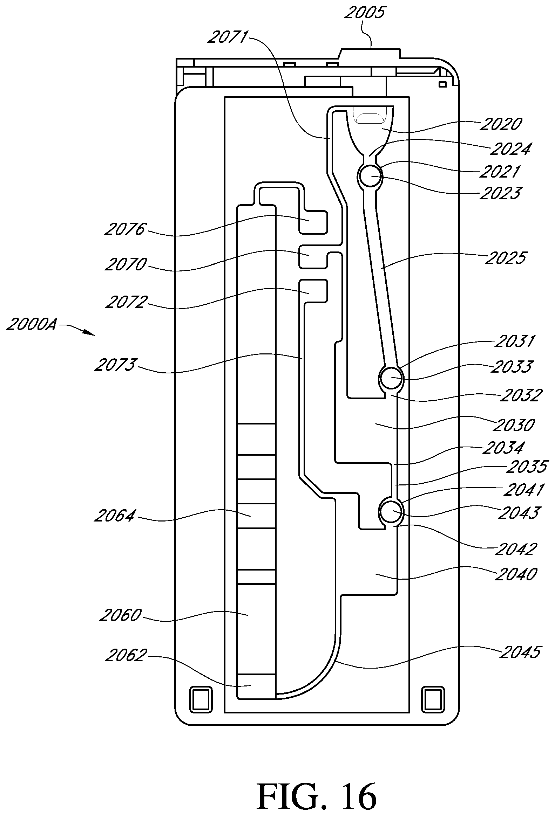

FIG. 16 is a plan view of an embodiment of fluidic component 2000A of fluidic device 2500 of FIG. 15. As depicted in FIG. 16, the fluidic network of fluidic component 2000A includes sample chamber 2020 which is in fluid communication with sample port 2005. In FIG. 16, proximal to sample chamber outlet 2024 is first reagent recess 2021 containing first reagent pellet 2023. As the sample passes through first reagent recess 2021, mixing of the sample with dried or lyophilized reagents contained therein is promoted. As previously described herein, effective mixing provides a uniform solution and thereby a uniform reaction mixture. As previously described herein for reagent pellet 1020 of FIG. 7, first reagent pellet 2023 can include a variety of reagents related to PLA analysis, such as, for example, but not limited by, antibody conjugate reagents. In a second non-limiting example, first reagent pellet 2023 can include reagents for sample pretreatment prior to processing a sample for PLA analysis, for example, for reagents for pH or ionic strength adjustment, or depending on the nature of the sample, a lysing reagent.

First fluidic channel 2025 is in fluid communication with first processing chamber 2030. Proximal to first processing chamber inlet 2032 is second reagent recess 2031 containing second reagent pellet 2033. As the sample passes through second reagent recess 2031, mixing of the sample with dried or lyophilized reagents contained therein is promoted, thereby ensuring a uniform reaction mixture. First processing chamber 2030 can be utilized to incubate a sample with an antibody conjugate reagent prior to preparation of protein-target-linked template generation and amplification as described for steps 110 and 120 of FIG. 7. Accordingly, reagents in either first reagent recess 2021 or second reagent recess 2031 are formulated with all necessary components required for the binding of a target protein if present in a sample with two antibody conjugates, each coupled to complementary oligonucleotide strands and each directed to a different epitope of a target protein as described for FIG. 2A through FIG. 2C. Accordingly, a dried or lyophilized antibody conjugate reagent pellet can contain buffering agents, salts, antibody conjugates, as well as stabilizing excipients, such as polyols, surfactants and antioxidants.

Second fluidic channel 2035 is proximal to first processing chamber outlet 2034 and is in fluid communication with second processing chamber 2040. Proximal to second sample processing chamber inlet 2042 is third reagent recess 2041 containing third reagent pellet 2043. Dried or lyophilized reagents present in third reagent recess 2041 can mix with fluid as it passes through the recess to second processing chamber 2040, thereby providing a uniform reaction mixture. Reagents in third reagent recess 2041 are formulated for reaction with the sample in second processing chamber 2040, which can combine template generation and amplification in a single chamber. Accordingly, a dried or lyophilized reagent pellet in third reagent recess 2041 can contain all components necessary for ligation, for example such as buffering agents, salts, ATP and a ligase enzyme, as well as all components necessary for the amplification reaction, for example such as buffering agents, salts, dNTPs, rNTPs, forward and reverse primers, as well as a heat stable polymerase. Additionally, a positive control such as a virus, bacteria or nucleic acid can be included in a dried or lyophilized formulation of amplification reagents in third reagent recess 2041.

Third fluidic channel 2045 is proximal to second processing chamber outlet 2034 and is in fluid communication with detection chamber 2060. As depicted in FIG. 15, detection chamber 2060 includes fluid compartment 2062 at the inlet end of detection chamber 2060 and lateral flow strip 2064. Fluid compartment 2062 provides a space of sufficient capacity to accommodate substantially all or the entire volume of fluid in the detection chamber at a height that enables the fluid to flow up the detection strip by capillary action without flooding and to enable correct capillary migration up the detection strip to ensure effective detection. As will be described in more detail here, fluid compartment 2062 can contain lyophilized detection reagents including detection probes.

Regarding fluidic flow control for fluidic component 2000A of FIG. 16, a sample introduced into sample port 2005 can flow to sample chamber 2020 and then to first processing chamber 2030, as first processing chamber 2030 is vented to an expansion volume (not shown) via vent channel 2071, which is in fluid communication with unsealed vent pocket 2070. Fluid movement from first processing chamber 2030 to second processing chamber 2040 is accomplished by actuating the opening of vent pocket 2072 connected to second processing chamber 2040 via vent channel 2073. When fluid enters first chamber processing chamber 2030, vent pocket 2072, connected to second processing chamber 2040, is sealed, and thus fluid will not pass through second fluidic channel 2035 connecting the two chambers. As will be described in more detail herein, rupture of a seal of vent pocket 2072 allows vent pocket 2072 and thus second processing chamber 2040 to be in fluid communicate with unsealed vent pocket 2070 and thereby with an expansion volume (not shown). In a similar fashion, fluid movement from second processing chamber 2040 to detection chamber 2060 is accomplished by actuating the opening of vent pocket 2076 connected to detection chamber 2060 via vent channel 2077. Rupture of a seal of vent pocket 2076 allows vent pocket 2076 and thus detection chamber 2060 to be in fluid communication with unsealed vent pocket 2070 and thereby with an expansion volume (not shown).This method of fluid movement embodied within a hermetically sealed expansion volume provides for containment of sample fluids within a fluidic device of the present disclosure, thereby containing biohazard associated with various biological samples within the fluidic device.

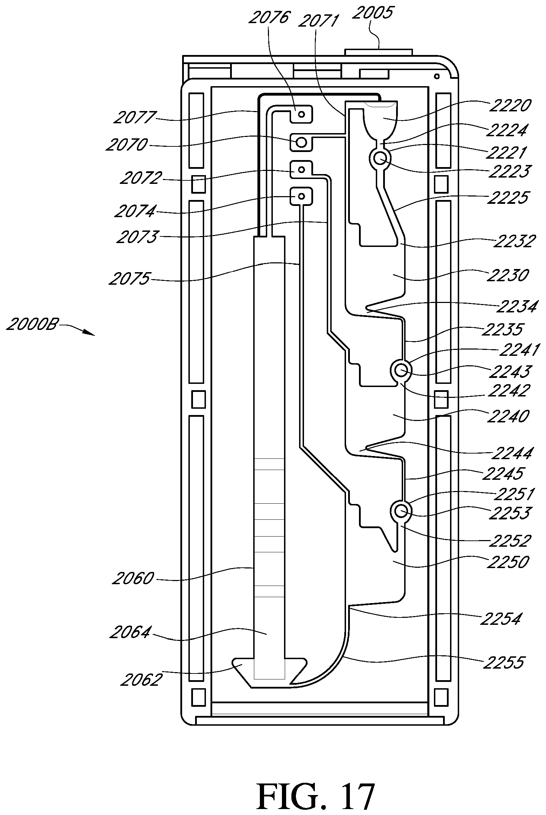

FIG. 17 is a plan view of an additional embodiment of fluidic component 2000B of fluidic device 2500 of FIG. 15. The fluidic network of fluidic component 2000B of FIG. 17 shares many similar features in comparison to the fluidic network of fluidic component 2000A of FIG. 16. The fluidic network of fluidic component 2000B of FIG. 17 additionally includes a third processing chamber, vent pocket and vent channel.

The fluidic network of fluidic component 2000B includes sample chamber 2220 which is in fluid communication with sample port 2005. In FIG. 17, proximal to sample chamber outlet 2224 is first reagent recess 2221 containing first reagent pellet 2223. As a sample passes through first reagent recess 2221, mixing of the sample with dried or lyophilized reagents contained therein is promoted. As previously described herein, effective mixing provides a uniform solution and thereby a uniform reaction mixture. As will be described in more detail herein, first reagent pellet 2223 can include a variety of reagents related to PLA analysis, such as reagents for sample pretreatment prior to processing a sample for PLA analysis or such as antibody conjugate reagents. First fluidic channel 2225 is in fluid communication with first processing chamber 2230, which includes first processing chamber inlet 2232 and first processing chamber outlet 2234. First processing chamber 2230 can be utilized to as for processing a sample for pretreatment or to incubate a sample with an antibody conjugate reagent prior to preparation of protein-target-linked template generation and amplification as described for steps 110 and 120 of FIG. 7.

Second fluidic channel 2235 is proximal to first processing chamber outlet 2234 and is in fluid communication with second processing chamber 2240. Proximal to second sample chamber inlet 2242 is second reagent recess 2241 containing second reagent pellet 2243. As a sample passes through second reagent recess 2241, mixing of the sample with dried or lyophilized reagents contained therein provides a uniform solution and thereby a uniform reaction mixture. As will be described in more detail herein, second processing chamber 2240 can be utilized for a variety of PLA processing steps. As such, second reagent recess 2241 can have a second reagent pellet 2243 varying on composition depending on what processing step is accommodated in second processing chamber 2240. For example, second reagent pellet 2243 can include reagents related to various processing steps of PLA analysis, such as antibody conjugate reagent, or ligation reagents or a combination of ligation and PCR reagents. Accordingly, processing in second processing chamber 2240 can include incubation of the sample with antibody conjugate reagents, incubation with ligation reagents, or incubation with a combination of ligation and PCR reagents.

Third fluidic channel 2245 is proximal to second processing chamber outlet 2244 and is in fluid communication with third processing chamber 2250. Proximal to third sample chamber inlet 2252 is third reagent recess 2251 containing third reagent pellet 2253. As a sample passes through third reagent recess 2251, mixing of the sample with dried or lyophilized reagents contained therein is provides a uniform solution and thereby a uniform reaction mixture. As will be described in more detail herein, third processing chamber 2250 can be utilized for a variety of PLA processing steps. As such, third reagent recess 2251 can have a third reagent pellet 2253 varying on composition depending on what processing step is accommodated in third processing chamber 2250. For example, third reagent pellet 2253 can include reagents related to various processing steps of PLA analysis, such PCR reagents, or a combination of ligation and PCR reagents or detection probe labeling reagents. Accordingly, processing in third processing chamber 2240 can include incubation of the sample with PCR reagents. incubation with a combination of ligation and PCR reagents or incubation with detection probe labeling reagents.

As previously described herein for fluidic component 2000B, dried or lyophilized sample pretreatment reagents can include reagents for pH or ionic strength adjustment, or depending on the nature of the sample, a lysing reagent. Dried or lyophilized antibody conjugate reagents contain two antibody conjugates, each coupled to complementary oligonucleotide strands and each directed to a different epitope of a target protein as described for FIG. 2A through FIG. 2C. Accordingly, a dried or lyophilized antibody conjugate reagent pellet can contain buffering agents, salts, antibody conjugates, as well as stabilizing excipients, such as polyols, surfactants and antioxidants. Dried or lyophilized ligation reagents can include buffering agents, salts, ATP and a ligase enzyme, while dried or lyophilized reagents containing both ligation and PCR reagents can contain buffering agents, salts, ATP and a ligase enzyme, as well as all components necessary for the amplification reaction, for example such as buffering agents, salts, dNTPs, rNTPs, forward and reverse primers, as well as a heat stable polymerase. Additionally, a positive control such as a virus, bacteria or nucleic acid can be included in a dried or lyophilized formulation including amplification reagents.

Various processes conducted in various chambers of fluidic component 2000B to carry out a PLA or PEA assay or both as described for method 100 of FIG. 1 can vary for various embodiments of fluidic device 2500 of FIG. 15 utilizing fluidic component 2000B.

By way of a first non-limiting example, first reagent pellet 2023 contained in first recess 2221 can include antibody conjugate reagents for forming a protein-antibody conjugate complex with a target protein if present in the sample, and incubation of the sample with the antibody conjugate reagents can be performed in first processing chamber 2230. Second reagent pellet 2243 formed in second reagent recess 2241 can contain ligation reagents for forming target-linked template nucleic acids, and incubation of the sample with the ligation reagents can be performed in second processing chamber 2240. Third reagent pellet 2253 contained in third reagent recess 2251 can contain PCR reagents for forming template nucleic acid amplicons, and amplification can be performed in third processing chamber 2250. As will be described in more detail herein, template nucleic acid amplicons formed in processing chamber 2250 can be labeled with detection probes in detection chamber 2060.

In a second non-limiting example, first reagent pellet 2023 contained in first recess 2221 can include reagents for sample pretreatment prior to processing a sample for PLA analysis, for example, for reagents for pH or ionic strength adjustment, or depending on the nature of the sample, a lysing reagent. Preprocessing of a sample can be performed in first processing chamber 2230. Second reagent pellet 2243 formed in second reagent recess 2241 can contain antibody conjugate reagents for forming a protein-antibody conjugate complex with a target protein if present in the sample, and incubation of the sample with the antibody conjugate reagents can be performed in second processing chamber 2240. Third reagent pellet 2253 contained in third reagent recess 2251 can contain ligation reagents for template nucleic acid formation, as well as PCR reagents for amplification, and processing to form template nucleic acids and template nucleic acid amplicons can be performed in processing chamber 2250. Template nucleic acid amplicons formed in processing chamber 2250 can be labeled with detection probes in detection chamber 2060.

In a third non-limiting example, first reagent pellet 2023 contained in first recess 2221 can include antibody conjugate reagents for forming a protein-antibody conjugate complex with a target protein if present in the sample, and incubation of the sample with the antibody conjugate reagents can be performed in first processing chamber 2230. Second reagent pellet 2243 contained in second reagent recess 2241 can contain ligation reagents for template nucleic acid formation, as well as PCR reagents for amplification, and processing to form template nucleic acids and template nucleic acid amplicons can be performed in processing chamber 2240. Third reagent pellet 2253 contained in third reagent recess 2251 can contain detection probe labeling reagents for labeling template nucleic acid amplicons, and labeling can be performed in processing chamber 2250. A sample with detection labeled amplicons formed in processing chamber can flow into fluid compartment 2062 of detection chamber 2060, and develop on lateral flow strip 2064.

In a forth non-limiting example, first reagent pellet 2023 contained in first recess 2221 can include antibody conjugate reagents for forming a protein-antibody conjugate complex with a target protein if present in the sample, and incubation of the sample with the antibody conjugate reagents can be performed in either first processing chamber 2230 or second processing chamber 2240. Third reagent pellet 2253 contained in third reagent recess 2251 can contain ligation reagents for template nucleic acid formation, as well as PCR reagents for amplification, and processing to form template nucleic acids and template nucleic acid amplicons can be performed in processing chamber 2250. Template nucleic acid amplicons formed in processing chamber 2250 can be labeled with detection probes in detection chamber 2060.

In all preceding non-limiting examples, forth fluidic channel 2255 is proximal to second processing chamber outlet 2254 and is in fluid communication with detection chamber 2060. As such, all fluidic network elements of detection chamber 2060 for fluidic component 2000B of FIG. 17 are the same as described for fluidic component 2000A of FIG. 16. As such, the present disclosure regarding labeling and detection is applicable to fluidic component 2000A of FIG. 16 and fluidic component 2000B of FIG. 17.

Fluid compartment 2062 can contain lyophilized detection reagents including detection probes. As previously described herein, a detection probe is a conjugate of a detection oligonucleotide that is complementary to a template nucleic acid or to a control and a detectable microsphere providing for visual and optical detection. Suitable detection probes include oligonucleotide conjugated to a detectable solid support, such as a polystyrene microspheres, latex beads or paramagnetic beads. Such detectable solid-supports can incorporate a range of detectable moieties including visible dyes, fluorescent dyes, quantum dots or colloidal gold. Accordingly, a dried or lyophilized detection reagent pellet can contain buffering agents, salts, and detection probes.

Additionally, fluid compartment 2062 provides a space of sufficient capacity to accommodate substantially all or the entire volume of fluid in the detection chamber at a height enabling flow up the detection strip, such as lateral flow substrate 2064 of FIG. 16 and FIG. 17, without flooding and ensuring correct capillary migration up the lateral flow detection strip to ensure effective detection. In that regard, for efficient labeling to occur, sample entering fluid compartment 2062 from third fluidic channel 2045 can mix with lyophilized detection reagents. Fluid compartment 2062 is configured to provide effective mixing and dispersion of particles to facilitate a uniform reaction mixture, as well as increasing the uniformity of particle migration on lateral flow substrate 2064. Fluid compartment 2062 of FIG. 16 and FIG. 17 can be especially advantageous for low volume assays, such as assays with a volume less than 200 µL, or more specifically less than between about 40 µL to about 100 µL in volume.

As previously described herein for a lateral flow substrate, such as lateral flow substrate 2064 of FIG. 16 and FIG. 17, capture probes can be contained in or on a lateral flow substrate, which can be a porous matrix. Such capture probes are concentrated at a location defining a test line extending transverse to the capillary flow direction through a lateral flow substrate. Capture probes can patterned on a lateral flow substrate in other shapes, for example dots, a plus sign, a minus sign, etc. Capture probes are configured to capture a detection oligonucleotide conjugated to a detection probe. When a sufficient level of labeled target template amplicons are captured, the concentration of detection probes along a test line or other pattern becomes visually and optically detectable. As such, sufficient concentration of detection probe immobilized by patterned capture probe on a lateral flow substrate provides an indication of presence of a detectable concentration of a target-linked template nucleic acid or control .

Regarding fluidic flow control for fluidic component 2000B of FIG. 17, the principles of actuated fluid flow control are the same as described for fluidic component 2000A of FIG. 16, with the exception that there is an additional vent pocket and vent pocket channel associated with third processing chamber 2250.

For fluidic component 2000B, a sample introduced into sample port 2005 can flow to sample chamber 2220 and then to first processing chamber 2230, as first processing chamber 2230 is vented to an expansion volume (not shown) via vent channel 2071, which is in fluid communication with unsealed vent pocket 2070. Fluid movement from first processing chamber 2230 to second processing chamber 2240 is accomplished by actuating the opening of vent pocket 2072 connected to second processing chamber 2240 via vent channel 2073. When fluid enters first processing chamber 2230, vent pocket 2072, connected to second processing chamber 2240, is sealed, and thus fluid will not pass through second fluidic channel 2235 connecting the two chambers. The rupture of a seal of vent pocket 2072 allows vent pocket 2072 and thus second processing chamber 2240 to be in fluid communicate with unsealed vent pocket 2070 and thereby with an expansion volume (not shown). For fluidic component 2000B, fluid movement from second processing chamber 2240 to third processing chamber 2250 is accomplished by actuating the opening of vent pocket 2074 connected to third processing chamber 2250 via vent channel 2075. Rupture of a seal of vent pocket 2074 allows vent pocket 2074 and thus processing chamber 2250 to be in fluid communicate with unsealed vent pocket 2070 and thereby with an expansion volume (not shown). Fluid movement from third processing chamber 2250 to detection chamber 2060 is accomplished by actuating the opening of vent pocket 2076 connected to detection chamber 2060 via vent channel 2077. Rupture of a seal of vent pocket 2076 allows vent pocket 2076 and thus detection chamber 2060 to be in fluid communicate with unsealed vent pocket 2070 and thereby with an expansion volume (not shown). As previously described herein for FIG. 16, this method of fluid movement provides for containment of sample fluids within a hermetically sealed expansion volume, thereby containing biohazard associated with various biological samples within the fluidic device.

In addition to a third processing chamber, other features of the fluidic network depicted for fluidic component 2000B of FIG. 17 provide for enhanced mixing and/or flow control through processing chambers of various described fluidic devices of the present teachings. Accordingly, exemplary features described in FIG. 18 through FIG. 20 are features that are applicable to fluidic component 1000 (FIG. 7) of fluidic device 1250 of FIG. 13, as well as fluidic component 2000A (FIG. 16) and fluidic component 2000B (FIG. 17) of fluidic device 2500 of FIG. 15.

FIG. 18 is an expanded view of exemplary reagent recess 2100 showing the inner volume of a portion of a fluidic network of the present disclosure including reagent recess 2100. As previously described herein, a reagent recess can be in fluid communication with a processing chamber, for example proximal to an inlet or outlet of a chamber. As such, a fluidic device of the present disclosure can have a plurality of reagent recesses in a fluidic network. As depicted in FIG. 18, fluidic channel 2125 is in fluid communication with reagent recess 2100. Reagent recess outlet 2122 is proximal to processing chamber 2100. Reagent recess 2100 can include structures on one or more of the surfaces facing a reagent pellet to assist with directing fluids, preferably using capillarity or surface tension effects to facilitate rehydration of the dried reagents. Such features may comprise ridges, such as ridges 2110 of FIG. 18, grooves, dimples or other structures enabling fluid flow to be directed to the internal space of a reagent recess as the fluid passes through the reagent recess.

FIG. 19 depicts exemplary processing chamber 2130 with triangular projection 2135, which can extend substantially horizontally into processing chamber 2130 when a fluidic device is oriented substantially vertically during use. As depicted in FIG. 19 inlet channel 2136 is in fluid communication with processing chamber 2130. Processing chamber includes processing chamber inlet 2132 and processing chamber outlet 2134, as well as first sidewall 2131 and second sidewall 2132. As depicted in FIG. 19, triangular projection 2135 is generally triangular in shape. The resulting flow path of the fluid has a horizontal component, thereby sufficiently increasing the effective length of the flow path without adding vertical spacing between the chamber and sufficiently decreasing the flow velocity of the fluid. Additionally, the generally triangular shape triangular projection 2135 provides processing chamber outlet 2134 with a taper.

As fluid enters processing chamber 2130 from fluidic inlet channel 2136, it flows toward processing chamber outlet 2134. Due to triangular projection 2135, flow is redirected towards sidewall 2133. As the flow proceeds to processing chamber corner 2137, the flow divides, with some entering processing chamber outlet 2134, while the rest contacts sidewall 2133 and is directed upward, creating a swirling effect which improves mixing in processing chamber 2130. During use of processing chamber 2130 for processing a sample, fluid passage below fluidic outlet channel 2138 proximal to processing chamber outlet 2134 is blocked. Additionally, the taper of processing chamber outlet 2134 increases compressible air volume at the entrance of the chamber. As the fluid enters processing chamber outlet 2134 at a lower velocity, reducing the distance the fluid flows down the tapered processing chamber outlet 2134 before it stops. The combination of decreased velocity of fluid flow and back pressure created by the taper ensures containment of fluid in processing chamber 2130 during sample processing in chamber 2130.

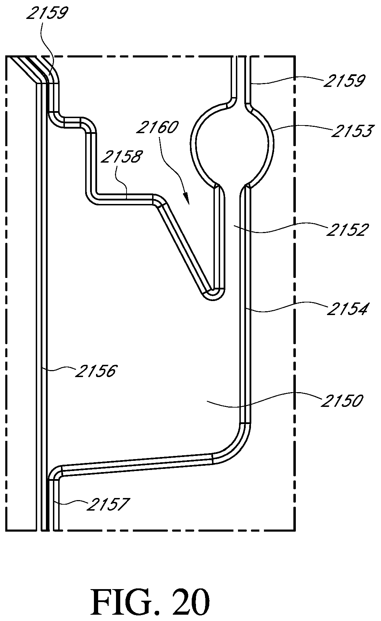

FIG. 20 depicts exemplary processing chamber 2150 with triangular flow control feature 2160, which can extend substantially vertically into processing chamber 2150 when a fluidic device is oriented substantially vertically during use. As depicted in FIG. 20, processing chamber 2150 can include first wall 2154, second wall 2156, and third wall 2158. Reagent recess 2153 is between fluidic inlet channel 2155 and processing chamber inlet channel 2152. Processing chamber inlet channel 2152 can be formed between first wall 2154 and third wall 2158; first outlet channel 2157 can be formed between first wall 2154 and second wall 2156, while second outlet channel 2159 can be formed between second wall 2156 and third wall 2158. Processing chamber inlet channel 2152 can be generally directed toward first outlet channel 2157 and generally directed away from second outlet channel 2159.

Third wall 2158 can include triangular projection 2160, which can extend between processing chamber inlet channel 2152 and second outlet channel 2159 and into the internal volume processing chamber 2150. Triangular projection 2160 is generally triangular in shape, having first side 2162 and second side 2164. As depicted in FIG. 20, first side 2162 of triangular projection 2160 extends substantially vertically and forms a side of processing chamber inlet channel 2152. Second side 2164 of triangular projection 2160 extends upward toward the top of the reaction chamber at an angle less than approximately 60 degrees from vertical, or less than approximately 45 degrees from vertical, or even less than approximately 30 degrees from vertical. The sharp vertical rise on the side of triangular projection 2160 directs fluid flow toward the outlet instead of across third wall 2158, thereby providing for uniform mixing of newly resuspended reagent from reagent recess 2153.

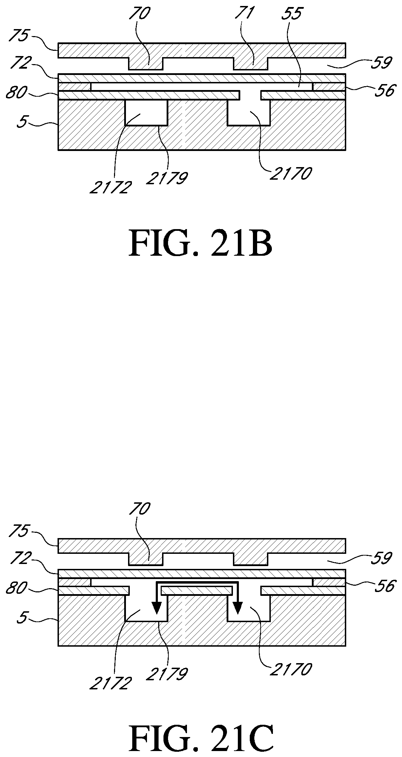

FIG. 21A and FIG. 21B are schematic representations illustrating how vent pocket opening to an internal volume provides fluidic flow control in a hermetically-sealed fluidic device. The present disclosure for FIG. 21A and FIG. 21B illustrates vent pocket opening for exemplary vent pockets 2170 and 2172, and are applicable for all vent pockets previously described for fluidic component 2000A of FIG. 16 and fluidic component 2000B of FIG. 17.

To enable a hermetically sealed fluidic device, selectively heat resistant and heat labile materials are layered in the manner represented schematically in cross section in FIG. 21A and FIG. 21B. Heating device 75, which can be a flexible circuit and/or a resistive heater on printed circuit board (PCA), provides heat sources 70 and 71, which are placed in register with vent pockets 2170 and 2172 and in proximity to heat labile vent pocket seal material layer 80. As depicted in FIG. 21A, vent pocket 2170 was previously ruptured by heat source 71, providing fluid communication of vent pocket 2170 with an expansion chamber (not shown). The vent pocket seal may comprise a heat labile material such as a polyolefin film or a polystyrene film, such as a biaxially oriented polystyrene (BOPS) film. Heat stable material layer 72, such as a polyimide film, is disposed between heat source 70 and heat labile vent pocket seal material 80 to form a hermetic barrier. In some embodiments sealed space 55 between or surrounding vent pockets is augmented by the inclusion of gasket or spacer 56 comprising an adhesive layer that bonds heat stable material 72 to heat labile material 80 and/or fluidic component 5 and maintains a hermetic seal of the fluidic device in the region of the vents after one or more of the vents are opened. Sealed space 55 can also provide an air gap for the communication of air between opened vent 2170 and an expansion volume (not shown).

As depicted in FIG. 21A and FIG. 21B, heat is transferred from heat source 70 through heat stable material 72 and sealed space 55 to the heat labile vent pocket seal material 80, rupturing it and opening vent pocket 2172. As previously described here with respect to vent-actuated flow control for fluidic component 2000A of FIG. 16 and fluidic component 2000B of FIG. 17, after rupture of heat labile vent pocket seal material 80, vent pocket 2172 is in fluid communication with open vent pocket 2170, and as such in fluid communication with a hermetically-sealed expansion volume such that fluid (i.e. gas, vapor, and liquid) within a fluidic device of the present disclosure may remain sealed with respect to the external environment, thereby containing biohazard associated with various biological samples within the fluidic device. Variations of a vent pocket of the present disclosure can utilize seals other than a heat-sensitive membrane, and can utilize other methods of breaking a seal, such as puncturing, tearing, or dissolving.

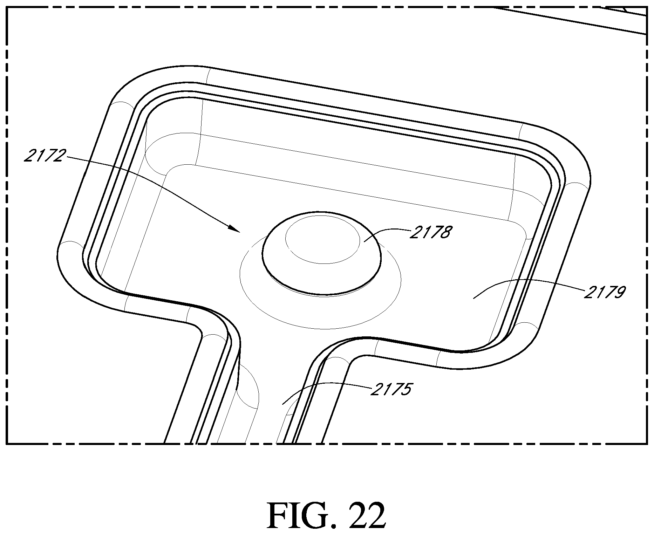

FIG. 22 depicts an exemplary vent pocket of the present disclosure. Vent pocket 2172 od FIG. 22 is cross-referenced to the schematic depiction of vent pocket 2172 of FIG. 21A. Vent pocket 2172 of FIG. 22 is in fluid communication with vent pocket channel 2175 and includes raised structure 2178 formed on vent pocket face 2179 (see also FIG. 21A and FIG. 21B). Raised structure 2178 can be a dimple, protrusion, asperity, or other similar structure to facilitate the formation of an opening during rupture of a heat-labile vent seal material, such as heat labile vent pocket seal material 80 of FIG. 21A. Additionally, such a structure can prevent resealing of a vent pocket. With reference to FIG. 21A, resealing of a vent pocket can occur during the heat-induced rupture of heat labile vent pocket seal material 80 in which heat stable material 72 can deform, pressing it against heat labile vent pocket seal material 80. Once the seal ruptures, the molten seal material can form a secondary seal with heat stable material 72, thereby closing the vent. Raised structure 2178 of FIG. 22; a dimple, protrusion, asperity, or other similar structure, can mitigate the occurrence of resealing.

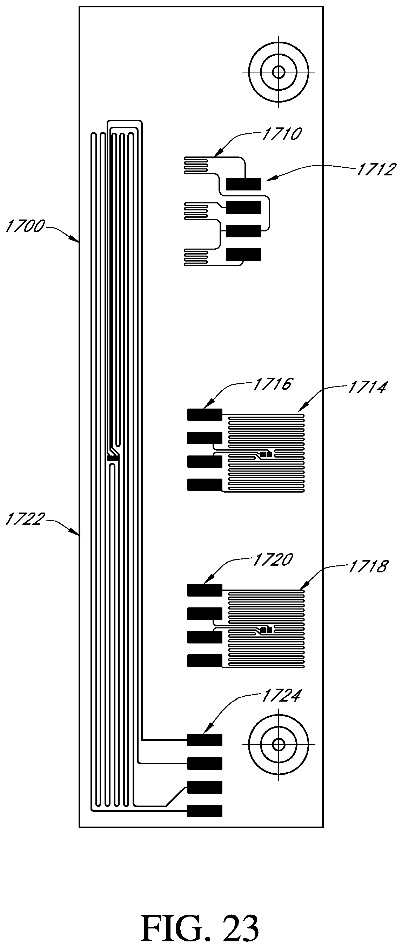

FIG. 23 is an embodiment of a flexible heater comprising resistive heating elements corresponding to various chambers and vent pockets of fluidic devices of the present disclosure. Flexible heater 1700 of FIG. 23 can be interfaced with fluidic component 2000A of FIG. 16 and fluidic component 2000B of FIG. 17. As depicted in FIG. 23, flexible heater 1700 is fabricated with resistive heating elements that are electrically coupled to a electrical pads that provide current to each corresponding resistive heating element. Flexible heater 1700 is fabricated in a heat stable material, such as polyimide. Each resistive element of flexible heater 1700 can be aligned to chamber or vent pocket that can be heated in accordance with an automated analysis work flow, such as depicted for FIG. 1. As such, resistive heating elements can be actuated in a controlled fashion to control, for example, fluid flow, cell lysis, thermal cycling, as well as controlling the temperature of the solution as it migrates up the detection strip.

For example, resistive heating elements 1710 can be aligned with vent pockets and utilized for opening a vent pocket as previously described herein with respect to FIG. 16, FIG. 17 and FIGs. 21A-21B. Electrical pads 1712 provide current to heating elements 1710. Similarly, resistive heating element 1714 associated with electrical pads 1716 and resistive heating elements 1718 associated with electrical pads 1720 can be aligned for heating each processing chamber. Finally, resistive heating element 1722 associated with electrical pads 1724 can be aligned with for heating a detection chamber. Direct contact between the flexible heater and the fluidic features to be thermally controlled by heaters on the flexible heater provides for a low thermal mass system capable of rapid temperature changes. To enable collection of temperature data for use in temperature regulation, a temperature sensor or sensors may be incorporated into the flexible heater. Additionally, as will be described herein in more detail, a non-contact of temperature monitoring device such as an infrared sensor may be employed, or a combination of on-flexible heater monitoring and remote monitoring can be utilized.

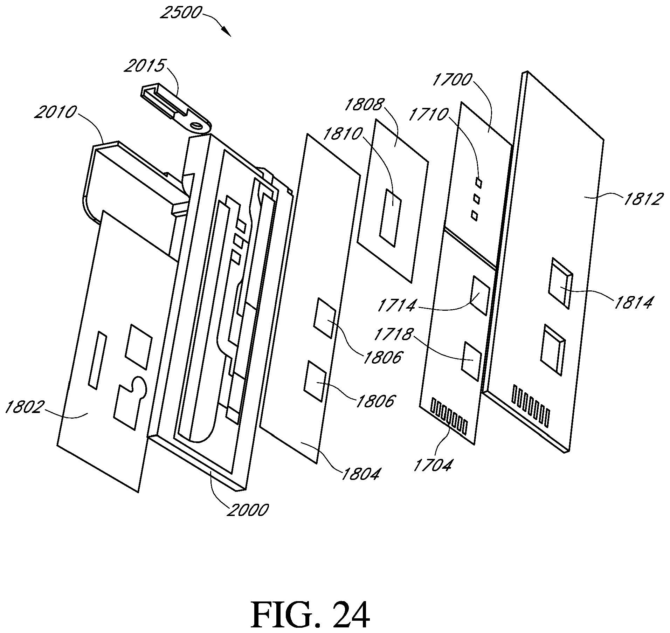

FIG. 24 is an exploded view of an embodiment of a fluidic device of the present disclosure, showing the respective components comprising fluidic device 2500 of FIG. 15. As shown in FIG. 24, fluidic device 2500 can include cover 2010 over which sliding seal 2015 is mounted as part, as well as front label 1802. The front of fluidic component 2000 (not shown) is oriented so the lateral flow strip can be seen through front cover 1802, while the fluidic network oriented towards flexible heater 1700 is interfaced with thermally labile film 1804. Windows 1806 in thermally labile film 1804, which can be a polystyrene material, such as bilaterally oriented polystyrene (BOPS), are depicted situated over processing chambers to allow direct contact of processing chambers with heating element 1714 and heating element 1718 of flexible heater 1700. Direct contact between the flexible heater 1700 and the processing chambers to be temperature controlled provides for a low thermal mass system capable of rapid temperature changes. In this embodiment flexible heater 1700 serves as a heat stable seal to maintain a hermetically sealed fluidic device, similar to heat stable material 72 described for FIG. 21A and FIG. 21B. Variations of fluidic device 2500 can include an additional heat stable layer (for example comprising polyimide) placed between flexible 1700 and rear panel 1812. Rear panel 1812 can be formed from a thin plastic material and is covers flexible heater 1700 to protect it during handling. Rear panel 1812 can include windows 1814,which are aligned over heating element 1714 and heating element 1718 of flexible heater 1700 to facilitate cooling and temperature monitoring. Electrical contact with controlling electronics of the docking unit, which will be described in more detail herein, can be provided by a set of electrical pads 1704, which can include an edge connector or connector pins such as spring loaded pins.

Spacer 1808 is assembled between vent resistive heating elements 1710 of flexible heater 1700 and thermally labile layer 1804. Air gap 1810 of spacer 1808 provides an expansion volume that ensures fluid movement through open vent pockets while maintaining a sealed fluidic device. Spacer 1808 is thick enough to provide a sufficient air gap to equilibrate pressures between open vents, but is also sufficiently thin so as not to substantially impact the interface between the heaters and the corresponding vent pockets or the sealing of the fluidic device by the heat stable material. The use of an expansion volume to reduce or equilibrate pressure within the sealed fluidic device ensures that pressure disequilibria do not result in unfavorable or premature solution movements within the fluidic device and that pressure accumulation does not adversely impact desired fluid movement, such as movement between chambers or through channels. This pressure control, i.e. the establishment of designated pressure distributions throughout the device, enables the system to work as designed regardless of atmospheric pressure. The expansion volume therefore enables controlled fluid movements which are dependent upon stable pressure within the system to be employed, and also enables use of a hermetically sealed fluidic device, thereby containing biohazard associated with various biological samples within the fluidic device.

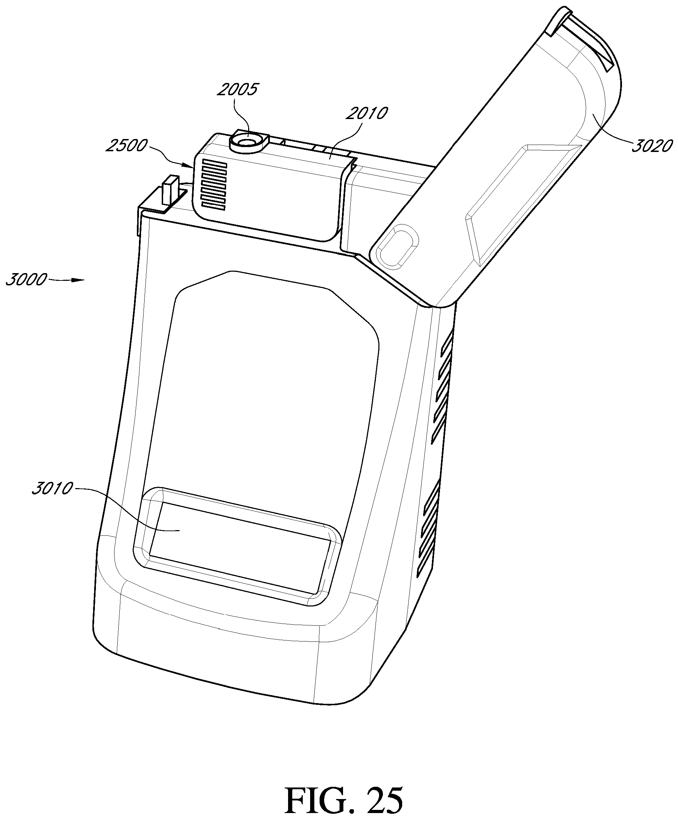

FIG. 25 depicts a front perspective view of a docking unit or dock of the present disclosure. Docking unit 3000 includes requisite electronic components to provide a fluidic device functionality, and is configured to interface with various fluidic devices of the present disclosure. A dock of the present disclosure can include electronic components to actuate all functions of a fluidic device required to run a test, as well providing an interface to an end user. Referring to FIG. 25, prior to sample addition by an end user, fluidic device 2500 is inserted into docking unit 3000. Display 3010 of docking unit 3000 can communicate information such as system function status, test protocols, test status to an end user. Display 3010 can be, for example, a LCD display, or a LED display or the like. Following fluidic device insertion into docking unit 3000, a test sample can be introduced to sample port 2005 of fluidic device 2500. An end user can initiate a test run by closing docking unit lid 3020. A mechanism (not shown) incorporated into the hinge of dock lid 3020 moves sliding seal 2015 (see FIG.15 and FIG. 24) of fluidic device 2500 to the closed position, thereby closing sample port 2005 during analysis.

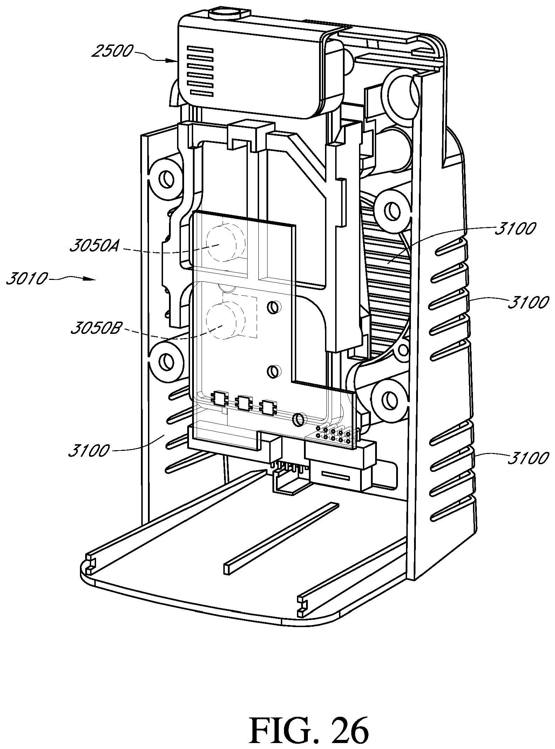

FIG. 26 depicts a front perspective view of the interior of a docking unit of the present disclosure, such as dock 3000 of FIG. 25, showing infrared temperature sensors 3050A and 3050B used to monitor the temperature of various processing chambers of fluidic device 2500. For example infrared sensors 2600 can be utilized to detect the temperature of processing chambers, such as processing chambers 2030 and 2040 of fluidic component 2000A and processing chambers 2030, 2040 and 2050 of fluidic component 2000B. Temperature sensors 3050A and 3050B enable the ongoing collection of temperature data for such processing chambers during a test run.

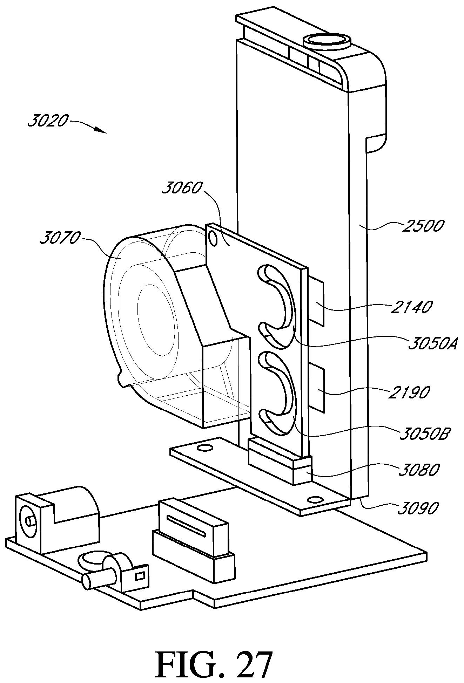

FIG. 27 depicts back perspective view 3020 the interior of a docking unit, such as dock 3000 of FIG. 25, showing infrared temperature sensors and other features of a docking unit of the present disclosure. As depicted in FIG. 27, infrared temperature sensors 3050A, and 3050B (see FIG. 26) are mounted on IR sensor support 3060, as to be aligned with processing chambers 2140 and 2190 of fluidic device 2500, respectively. Cooling of fluidic device 2500 can be provided by cooling fan 3070, which can be, for example, a muffin style fan. Additionally, a system of vents (see FIG. 26, reference number 3100) can be utilized to direct cooler outside air against the heat generated by the system and direct it out the sides of the device. When the IR assembly and fluidic device components are mated via temperature sensor assembly connector 3080 and fluidic device connector 3090, fluidic device 2500 is positioned in the docking unit in a substantially vertical or near-vertical orientation. An IR sensor board PCA can mounted some small distance from the opposite side of the fluidic device and can used for monitoring the heated processing chamber temperatures. This allows closed loop temperature control of the heating and cooling process, and accommodates ambient temperature variations.

### EXAMPLES:

### Overview of Analysis of Interferon Gamma-Induced Protein 10 (IP-10)

Interferon gamma-induced protein 10, IP-10, can be used to monitor kidney transplant rejection. For point-of-care testing (POCT), a detection level of about 10-15 pg/ml in urine is required. The present inventors undertook development of a conventional lateral flow assay of IP-10 in urine based on a sandwich assay with visual detection using colored microbead conjugates. The assay had a detection limit of greater than 100 pg/ml and required dilution of urine to prevent inconsistencies in results due to interferants in neat urine samples.

Preliminary work on PLA analysis of IP-10 using a conventional qPCR assay (ProQuantum Immunoassay Kit -Thermo Fisher catalog # RP-8647) showed good recovery of IP-10 from undiluted urine with and without spiked IP-10, as summarized in Table 1. In the PCR format, both recovery, as well as very low background for undiluted urine demonstrated improvement over the conventional lateral flow assay.

| ID | Measured conc. (ng/ml) | % Recovery |
|---|---|---|
| Urine Neat | 0.18 | N/A |
| Urine 1:2 | 0.11 | N/A |
| Urine 1:4 | 0.06 | N/A |
| Urine 1:8 | 0.04 | N/A |
| Urine Neat (62.5 pg/mL) | 56.11 | 89.8% |
| Urine 1:2 (62.5 pg/mL) | 58.48 | 93.6% |
| Urine 1:4 (62.5 pg/mL) | 60.00 | 96.0% |
| Urine 1:8 (62.5 pg/mL) | 61.88 | 99.0% |
| Urine Neat (12.5 pg/mL) | 10.67 | 85.4% |
| Urine 1:2 (12.5 pg/mL) | 12.51 | 100.1% |
| Urine 1:4 (12.5 pg/mL) | 11.95 | 95.6% |
| Urine 1:8 (12.5 pg/mL) | 9.94 | 79.5% |

However, during initial efforts focused on adapting the PLA analysis to a lateral flow format, the present inventors were confronted with a challenge of untoward precipitation in the sample processing chamber after heat denaturation of ligase. In a macro format, for example, in test tube, microfuge tube or PCR plate format, in a fully-equipped laboratory with trained personnel, precipitation during an analysis workflow can be readily addressed through, for example, filtration or centrifugation. In contrast, such precipitation in a sample-in-answer-out microdevice format can result in inconsistent results, and moreover may result in failure, for example, if occlusion of a fluidic component occurs due to blockage by precipitate.

Given the complex nature of urine as a sample, and given that that interferants in urine samples were problematic for the conventional IP-10 lateral flow assay, an extensive troubleshooting process was undertaken by the present inventors to understand what factors contributed to the formation of precipitates in the PLA lateral flow assay. The troubleshooting identified several sources contributing to precipitation, for example, the antibody conjugates, as well as the antibody dilution buffer and assay dilution buffer, which included non-reactive protein additives used for stabilization of assay components. A differentiating feature of incorporation of PLA into a microdevice format is that in addition to the analyte being a protein, there is substantially more protein in the assay format than would be included in various analyses in which the analyte is nucleic acid-based.

As such, as will be subsequently described in more detail herein, the present inventors have discovered how to adapt PLA analysis of proteins into a lateral flow format.

### METHOD FOR ANALYSIS OF INTERFERON GAMMA-INDUCED PROTEIN 10 (IP-10):

### Preparation of Reagents:

Antibody conjugates for IP-10 from ProQuantum Immunoassay Kit (Thermo Fisher catalog # RP-8647) were used with modification as will be described. Given that the antibody conjugates are an additional protein source, experiments were conducted to determine whether or not a minimum concentration of the conjugates providing an effective amount of oligonucleotide tag for amplification could be determined that, in conjunction with other adjustments to the reagents and workflow, also minimized protein precipitation. The antibody conjugate reagents were diluted in a phosphate buffered saline (PBS) antibody dilution buffer. The antibody dilution buffer was prepared in DNase/RNase free water and included non-reactive bulking proteins, for example, such as about 0.04 mg/ml of IgG (caprine) and 0.1% gelatin (piscine).

In order to utilize a single processing compartment of a lateral flow test device for ligation and PCR , such as described for chamber 1030 and chamber 1040 of FIG. 7-FIG. 14 for fluidic component 1000 of fluidic device 1250, a combined ligase/amplification/positive control reagent was prepared. As ligase is an additional protein source, in combination with optimizing the antibody conjugate concentration, optimization of ligase concentration from the kit was done to determine whether or not a minimum concentration of ligase providing effective ligation could be determined that minimized protein precipitation in subsequent processing steps. The ligase/amplification reagent was prepared using an assay dilution buffer. The assay dilution buffer was prepared in DNase/RNase free water and included non-reactive bulking proteins, for example, such as about 5% laboratory grade skim milk powder (bovine) and about 1% gelatin (piscine). Though the primers, the splint, and the PCR master mix from the kit were used, experimentation to adjust the PCR cycling conditions was done in conjunction with optimizing the concentrations of antibody conjugates and ligase.

As will be described in more detail subsequently herein, an antibody conjugate reagent and a ligase/amplification/positive control reagent effective in yielding an unexpectedly low visual detection for IP-10 in lateral flow format, while minimizing protein precipitation was determined. The conditions providing the unexpectedly low visual detection included antibody conjugates at 0.5x the concentration provided in the kit, and ligase at 0.25X the concentration provided in the kit in conjunction with a thermocycling protocol, which included cycling between 95°C for 5 seconds, followed by 60°C for 30 seconds with completion of thermocycling achieved after 30 cycles. Additionally, the positive control was a 141 nucleotide (nt) MS2 ultramer in final concentration of 1 pM, and also included the associated primers.

Test samples solutions of 1 pg/ml and 10 pg/ml of IP-10 synthetic analyte (SA) provided with the kit were prepared in a phosphate buffered saline (PBS) assay dilution buffer.

### IP-10 PLA Analysis in Lateral Flow Device:

Fluidic devices, such as described for FIGS. 7-13 for fluidic device 1250 or for fluidic device 2250 of FIGS. 15-24 were used. For each test sample run, a fluidic device was placed in a docking unit, such as described for FIG. 14 for fluidic device 1250 or as described for FIGS. 25-27 for fluidic device 2250.

Test samples were analyzed using the 1 pg/ml and 10 pg/ml IP-10 solutions. A sample of assay buffer was used to as a control for background. 60 µL of the sample solution was added to the sample chamber, and was combined with 5 µl of the antibody conjugate solution. The mixture was allowed to incubate at room temperature for 30 minutes. Upon actuation to open a first vent, the sample/antibody conjugate mixture passed into a sample processing chamber, such as chamber 1030 or chamber 1040 of FIG. 7 and was combined with 5 µl of the ligase/amplification reagent including the positive control. The mixture was allowed to incubate at room temperature in the processing chamber for 10 minutes to provide for adequate ligation of the oligonucleotide portions of the antibody reagents with the splint, followed by ligase inactivation at 95°C for 2 minutes. After the inactivation of ligase, 30 cycles of PCR was conducted in the PCR chamber, which took about 1 hour.

Upon completion of amplification, the reaction mixture reconstituted lyophilized detection particles as it flowed into the detection chamber. The lateral flow strip was allowed to develop for 5-10 minutes prior to visual interpretation of the results. Two capture lines were observed to be immobilized on the lateral flow membrane for the 1 pg/ml and 10 pg/ml test samples. From the bottom of the device, the first capture line was for a capture probe complementary to the amplification product of IP-10; and the second capture line was for a oligonucleotide complementary to the amplification product of the MS2 control. For the 0 pg/ml background control, only a single capture line for the MS2 control was observed.

Accordingly, by reducing protein precipitation through careful optimizing reagents to minimize total protein concentration in combination with adjusting thermocycling conditions, visual detection of IP-10 an order of magnitude below the target range of 10-15 pg/ml was observed.

Given the success in adaptation of PLA to IP-10, experimentation was conducted to determine whether or not additional proteins of clinical significance could also be adapted to a lateral flow format, such as described for fluidic device 1250 of FIG. 13 utilizing fluidic component 1000 of FIG. 7 or as described for fluidic device 2250 of FIG. 150 utilizing fluidic component 2000A of FIG. 16 and 2000B of FIG. 17.

### Method for Analysis of Human Epidermal Growth Factor (HuEGF)

Circulating levels of human epidermal growth factor (HuEGF) may be associated, for example, with end-stage renal disease, as well as being implicated in acute renal allograft rejection. Clinical levels of HuEGF for diagnostic or prognostic purposes have not yet been established.

### Preparation of Reagents:

Antibody conjugates for HuEGF from ProQuantum Immunoassay Kit (Thermo Fisher catalog # A35579) were used with modification as previously described. Initial conditions used included antibody conjugates at 0.5x the concentration provided in the kit, and ligase at 0.25X the concentration provided in the kit in conjunction with a thermocycling protocol, which included cycling between 95°C for 5 seconds, followed by 60°C for 30 cycles. The positive control was a 43 nucleotide (nt) ultramer in final concentration of 1 pM, and also included the associated primers.

Though a broad dynamic assay range up to 5000 pg/ml for HuEGF has been established for the PLA qPCR assay, detection level at the lower concentrations were of interest for the purpose of determining whether or not assay sensitivity in a lateral flow format could be established. Test samples solutions of 1.6 pg/ml and 8 pg/ml of HuEGF synthetic analyte (SA) provided with the kit were prepared in a phosphate buffered saline (PBS) assay dilution buffer.

### Hu EGF PLA Analysis in Lateral Flow Device:

Fluidic devices, such as described for FIGS. 7-13 for fluidic device 1250 or for fluidic device 2250 of FIGS. 15-24 were used. For each test sample run, a fluidic device was placed in a docking unit, such as described for FIG. 14 for fluidic device 1250 or as described for FIGS. 25-27 for fluidic device 2250.

Test samples were analyzed using the 1.6 pg/ml and 8 pg/ml HuEGF solutions. A sample of assay buffer was used to as a control for background. 60 µL of the sample solution was added to the sample chamber, and was combined with 5 µl of the antibody conjugate solution. The mixture was allowed to incubate at room temperature for 30 minutes. Upon actuation to open a first vent, the sample/antibody conjugate mixture passed into a sample processing chamber, such as chamber 1030 or chamber 1040 of FIG. 7 and was combined with 5 µl of the ligase/amplification reagent including the positive control. The mixture was allowed to incubate at room temperature in the processing chamber for 10 minutes to provide for adequate ligation of the oligonucleotide portions of the antibody reagents with the splint, followed by ligase inactivation at 95°C for 2 minutes. After the inactivation of ligase, 30 cycles of PCR was conducted in the PCR chamber.

Upon completion of amplification, the reaction mixture reconstituted lyophilized detection particles as it flowed into the detection chamber. The lateral flow strip was allowed to develop for 5-10 minutes prior to visual interpretation of the results. Two capture lines were observed to be immobilized on the lateral flow membrane for the 1.6 pg/ml and 8 pg/ml test samples. From the bottom of the device, the first capture line was for a capture probe complementary to the amplification product of HuEGF; and the second capture line was for a oligonucleotide complementary to the amplification product of the 43 nt ultramer control.

It should be noted that though a single capture line for the 43 nt ultramer control was observed for the background control, there was there was a high background on the lateral flow membranes strips. For clarity of interpretation of a background control at 0 pg/ml of analyte, it is desirable to eliminate background. As such, though the initial results are encouraging with respect to clear capture lines formed for the 1.6 pg/ml and 8 pg/ml test samples and positive control, efforts to trouble shoot the cause of the high background are continuing.

### Method for Analysis of Macrophage Inflammatory Protein-1 alpha (MIP-1α)

Circulating levels of macrophage inflammatory protein-1 alpha (MIP-1α) are implicated, for example, with graft rejection. Clinical levels of MIP-1α for diagnostic or prognostic purposes have not yet been established.

### Preparation of Reagents:

Antibody conjugates for MIP-1α from ProQuantum Immunoassay Kit (Thermo Fisher catalog # A35616) were used with modification as previously described. Initial conditions used included antibody conjugates at 0.5x the concentration provided in the kit, and ligase at 0.25X the concentration provided in the kit in conjunction with a thermocycling protocol, which included cycling between 95°C for 5 seconds, followed by 60°C for 30 cycles. The positive control was the same 43 nucleotide (nt) ultramer and associated primers used for the HuEGF assay, and was also used at a final concentration of 1 pM.

Though a broad dynamic assay range up to 10,000 pg/ml for MIP-1α has been established for the PLA qPCR assay, detection level at the lower concentrations were of interest for the purpose of determining whether or not assay sensitivity in a lateral flow format could be established. Test samples solutions of 16 pg/ml and 80 pg/ml of MIP-1α synthetic analyte (SA) provided with the kit were prepared in a phosphate buffered saline (PBS) assay dilution buffer.

### MIP-1α PLA Analysis in Lateral Flow Device:

Fluidic devices, such as described for FIGS. 7-13 for fluidic device 1250 or for fluidic device 2250 of FIGS. 15-24 were used. For each test sample run, a fluidic device was placed in a docking unit, such as described for FIG. 14 for fluidic device 1250 or as described for FIGS. 25-27 for fluidic device 2250.

Test samples were analyzed using the 16 pg/ml and 80 pg/ml MIP-1α solutions. A sample of assay buffer was used to as a control for background. 60 µL of the sample solution was added to the sample chamber, and was combined with 5 µl of the antibody conjugate solution. The mixture was allowed to incubate at room temperature for 30 minutes. Upon actuation to open a first vent, the sample/antibody conjugate mixture passed into a sample processing chamber, such as chamber 1030 or chamber 1040 of FIG. 7 and was combined with 5 µl of the ligase/amplification reagent including the positive control. The mixture was allowed to incubate at room temperature in the processing chamber for 10 minutes to provide for adequate ligation of the oligonucleotide portions of the antibody reagents with the splint, followed by ligase inactivation at 95°C for 2 minutes. After the inactivation of ligase, 30 cycles of PCR was conducted in the PCR chamber.

Upon completion of amplification, the reaction mixture reconstituted lyophilized detection particles as it flowed into the detection chamber. The lateral flow strip was allowed to develop for 5-10 minutes prior to visual interpretation of the results. Two capture lines were observed to be immobilized on the lateral flow membrane for the 16 pg/ml and 80 pg/ml test samples. From the bottom of the device, the first capture line was for a capture probe complementary to the amplification product of MIP-1α; and the second capture line was for a oligonucleotide complementary to the amplification product of the MS2 control. For the 0 pg/ml background control, only a single capture line for the MS2 control was observed.

Accordingly, the adaptation of PLA protein assays to a lateral flow format has been demonstrated using an effective strategy of optimizing reagents and protocols to minimize protein precipitation in a microfluidic lateral flow device.

Although the disclosure has been described in detail with particular reference to the described embodiments and examples, other embodiments can achieve the same or similar results. The specification and examples are to be considered exemplary only and are not limiting to the claims.

## Claims

1. A method of analyzing a sample for presence of a target protein, the method comprising:
processing the sample, the processing generating a first product, the first product comprising a template nucleic acid in response to the target protein being present in the sample, wherein processing the sample comprises contacting the sample with one or more reagents configured for performing a proximity ligation assay, or a proximity extension assay, or both;
contacting the first product with a detection probe, the contacting generating a second product, the detection probe configured to hybridize to the template nucleic acid to form a labeled nucleic acid; and
contacting a lateral flow substrate with the second product, the lateral flow substrate comprising a capture probe configured to hybridize to the labeled nucleic acid.

2. The method of claim 1, further comprising:
analyzing the lateral flow substrate; and
based on the analyzing, determining whether or not the sample comprises the target protein.

3. The method of claim 2, wherein analyzing comprises observing the lateral flow substrate for a detectable signal in response to hybridization of the labeled nucleic acid to the capture probe.

4. The method of claim 3, wherein the detectable signal is a colorimetric indicator on the lateral flow substrate and the observing comprises visually observing the lateral flow substrate.

5. The method of claim 3, wherein the detectable signal is fluorescence and the observing comprises using a sensor to detect the fluorescence.

6. The method of any one of claims 1-5, wherein the target protein is present in the sample at a concentration of less than 1.0 ng/mL.

7. The method of any one of claims 1-6, wherein processing the sample comprises contacting the sample with a first proximity probe and a second proximity probe, the first proximity probe having affinity for a first epitope of the target protein and the second proximity probe having affinity for a second epitope of the target protein.

8. The method of claim 7, wherein the first proximity probe comprises a first oligonucleotide and the second proximity probe comprises a second oligonucleotide.

9. The method of claim 7 or 8, wherein processing the sample further comprises contacting the sample with a third oligonucleotide configured to hybridize to both the first and second oligonucleotides.

10. The method of any one of claims 7-9, wherein the first proximity probe further comprises a first antibody having affinity for the first epitope, and the second proximity probe further comprises a second antibody having affinity for the second epitope, or wherein the first proximity probe further comprises a first antibody fragment having affinity for the first epitope, and the second proximity probe further comprises a second antibody fragment having affinity for the second epitope.

11. A method of detecting target protein in a sample, the method comprising:
forming a protein complex comprising a target protein, a first oligonucleotide, and a second oligonucleotide;
producing a template nucleic acid from the first and second oligonucleotides;
amplifying the template nucleic acid, the amplifying generating amplified nucleic acid;
labeling the amplified nucleic acid with a detection probe that hybridizes to the amplified nucleic acid, the labeling generating a labeled nucleic acid;
contacting the labeled nucleic acid with a lateral flow substrate, the lateral flow substrate comprising a capture probe configured to hybridize to the labeled nucleic acid; and
detecting a signal from the lateral flow substrate in response to hybridization of the labeled nucleic acid to the capture probe.

12. The method of claim 11, wherein the lateral flow substrate is part of a fluidic device.

13. The method of claim 12, wherein forming the protein complex, producing the template nucleic acid, and amplifying the template nucleic acid occur in a same chamber of the fluidic device, the chamber being upstream of the lateral flow substrate.

14. The method of claim 12, wherein labeling occurs at a location of the fluidic device that houses the lateral flow substrate.

15. The method of claim 12, wherein detecting comprises optical detection of the lateral flow substrate using a sensor.

## Patentansprüche

1. Verfahren zur Analyse einer Probe auf das Vorhandensein eines Zielproteins, wobei das Verfahren umfasst:
Verarbeiten der Probe, wobei die Verarbeitung ein erstes Produkt erzeugt, wobei das erste Produkt eine Template-Nukleinsäure als Reaktion auf das Vorhandensein des Zielproteins in der Probe umfasst, wobei das Verarbeiten der Probe das Kontaktieren der Probe mit einem oder mehreren Reagenzien umfasst, die für die Durchführung eines Proximity-Ligation-Assays oder eines Proximity-Extension-Assays oder beider konfiguriert sind;
Kontaktieren des ersten Produkts mit einer Detektionssonde, wobei das Kontaktieren ein zweites Produkt erzeugt, wobei die Detektionssonde konfiguriert ist, um an die Template-Nukleinsäure zu hybridisieren, um eine markierte Nukleinsäure zu bilden; und
Kontaktieren eines Lateral-Flow-Substrats mit dem zweiten Produkt, wobei das Lateral-Flow-Substrat eine Einfangsonde umfasst, die konfiguriert ist, um an die markierte Nukleinsäure zu hybridisieren.

2. Verfahren nach Anspruch 1, ferner umfassend:
Analysieren des Lateral-Flow-Substrats; und
basierend auf der Analyse, Bestimmen, ob die Probe das Zielprotein umfasst oder nicht.

3. Verfahren nach Anspruch 2, wobei das Analysieren das Beobachten des Lateral-Flow-Substrats auf ein detektierbares Signal als Reaktion auf die Hybridisierung der markierten Nukleinsäure an die Einfangsonde umfasst.

4. Verfahren nach Anspruch 3, wobei das detektierbare Signal ein kolorimetrischer Indikator auf dem Lateral-Flow-Substrat ist und das Beobachten das visuelle Beobachten des Lateral-Flow-Substrats umfasst.

5. Verfahren nach Anspruch 3, wobei das detektierbare Signal Fluoreszenz ist und das Beobachten das Verwenden eines Sensors zum Detektieren der Fluoreszenz umfasst.

6. Verfahren nach einem der Ansprüche 1 bis 5, wobei das Zielprotein in der Probe in einer Konzentration von weniger als 1,0 ng/ml vorhanden ist.

7. Verfahren nach einem der Ansprüche 1 bis 6, wobei das Verarbeiten der Probe das Kontaktieren der Probe mit einer ersten Näherungssonde und einer zweiten Näherungssonde umfasst, wobei die erste Näherungssonde Affinität für ein erstes Epitop des Zielproteins aufweist und die zweite Näherungssonde Affinität für ein zweites Epitop des Zielproteins aufweist.

8. Verfahren nach Anspruch 7, wobei die erste Näherungssonde ein erstes Oligonukleotid umfasst und die zweite Näherungssonde ein zweites Oligonukleotid umfasst.

9. Verfahren nach Anspruch 7 oder 8, wobei die Verarbeitung der Probe ferner das Kontaktieren der Probe mit einem dritten Oligonukleotid umfasst, das konfiguriert ist, um sowohl mit dem ersten als auch dem zweiten Oligonukleotid zu hybridisieren.

10. Verfahren nach einem der Ansprüche 7 bis 9, wobei die erste Näherungssonde ferner einen ersten Antikörper mit Affinität für das erste Epitop umfasst und die zweite Näherungssonde ferner einen zweiten Antikörper mit Affinität für das zweite Epitop umfasst, oder wobei die erste Näherungssonde ferner ein erstes Antikörperfragment mit Affinität für das erste Epitop umfasst und die zweite Näherungssonde ferner ein zweites Antikörperfragment mit Affinität für das zweite Epitop umfasst.

11. Verfahren zum Detektieren eines Zielproteins in einer Probe, wobei das Verfahren umfasst:
Bilden eines Proteinkomplexes, der ein Zielprotein, ein erstes Oligonukleotid und ein zweites Oligonukleotid umfasst;
Erzeugen einer Template-Nukleinsäure aus dem ersten und dem zweiten Oligonukleotid;
Amplifizieren der Template-Nukleinsäure, wobei das Amplifizieren amplifizierte Nukleinsäure erzeugt;
Markierung der amplifizierten Nukleinsäure mit einer Detektionssonde, die an die amplifizierte Nukleinsäure hybridisiert, wobei die Markierung eine markierte Nukleinsäure erzeugt;
Kontaktieren der markierten Nukleinsäure mit einem Lateral-Flow-Substrat, wobei das Lateral-Flow-Substrat eine Einfangsonde umfasst, die konfiguriert ist, um mit der markierten Nukleinsäure zu hybridisieren; und
Detektieren eines Signals vom Lateral-Flow-Substrat als Reaktion auf die Hybridisierung der markierten Nukleinsäure an die Einfangsonde.

12. Verfahren nach Anspruch 11, wobei das Lateral-Flow-Substrat Teil einer Fluidvorrichtung ist.

13. Verfahren nach Anspruch 12, wobei das Bilden des Proteinkomplexes, das Produzieren der Template-Nukleinsäure und das Amplifizieren der Template-Nukleinsäure in derselben Kammer der Fluidvorrichtung erfolgen, wobei die Kammer stromaufwärts des Lateral-Flow-Substrats liegt.

14. Verfahren nach Anspruch 12, wobei die Markierung an einer Stelle der Fluidvorrichtung erfolgt, die das Lateral-Flow-Substrat beherbergt.

15. Verfahren nach Anspruch 12, wobei das Detektieren eine optische Detektion des Lateral-Flow-Substrats unter Verwendung eines Sensors umfasst.

## Revendications

1. Procédé d'analyse d'un échantillon pour détecter la présence d'une protéine cible, le procédé comprenant :
le traitement de l'échantillon, le traitement générant un premier produit, le premier produit comprenant un acide nucléique matrice en réponse à la présence de la protéine cible dans l'échantillon, dans lequel le traitement de l'échantillon comprend la mise en contact de l'échantillon avec un ou plusieurs réactifs conçus pour effectuer un essai de ligature de proximité ou un essai d'extension de proximité, ou les deux ;
la mise en contact du premier produit avec une sonde de détection, la mise en contact générant un second produit, la sonde de détection étant configurée pour s'hybrider à l'acide nucléique matrice afin de former un acide nucléique marqué ; et
la mise en contact d'un substrat d'écoulement latéral avec le second produit, le substrat d'écoulement latéral comprenant une sonde de capture configurée pour s'hybrider à l'acide nucléique marqué.

2. Procédé selon la revendication 1, comprenant en outre :
l'analyse du substrat d'écoulement latéral ; et
en fonction de l'analyse, le fait de déterminer si l'échantillon comprend ou non la protéine cible.

3. Procédé selon la revendication 2, dans lequel l'analyse comprend l'observation du substrat d'écoulement latéral pour déceler un signal détectable en réponse à l'hybridation de l'acide nucléique marqué à la sonde de capture.

4. Procédé selon la revendication 3, dans lequel le signal détectable est un indicateur colorimétrique sur le substrat d'écoulement latéral, et l'observation comprend l'observation visuelle du substrat d'écoulement latéral.

5. Procédé selon la revendication 3, dans lequel le signal détectable est une fluorescence, et l'observation comprend l'utilisation d'un capteur pour détecter la fluorescence.

6. Procédé selon l'une quelconque des revendications 1 à 5, dans lequel la protéine cible est présente dans l'échantillon à une concentration inférieure à 1,0 ng/ml.

7. Procédé selon l'une quelconque des revendications 1 à 6, dans lequel le traitement de l'échantillon comprend la mise en contact de l'échantillon avec une première sonde de proximité et une seconde sonde de proximité, la première sonde de proximité ayant une affinité pour un premier épitope de la protéine cible et la seconde sonde de proximité ayant une affinité pour un second épitope de la protéine cible.

8. Procédé selon la revendication 7, dans lequel la première sonde de proximité comprend un premier oligonucléotide et la seconde sonde de proximité comprend un deuxième oligonucléotide.

9. Procédé selon la revendication 7 ou 8, dans lequel le traitement de l'échantillon comprend en outre la mise en contact de l'échantillon avec un troisième oligonucléotide conçu pour s'hybrider à la fois aux premier et deuxième oligonucléotides.

10. Procédé selon l'une quelconque des revendications 7 à 9, dans lequel la première sonde de proximité comprend en outre un premier anticorps ayant une affinité pour le premier épitope, et la seconde sonde de proximité comprend en outre un second anticorps ayant une affinité pour le second épitope, ou dans lequel la première sonde de proximité comprend en outre un premier fragment d'anticorps ayant une affinité pour le premier épitope, et la seconde sonde de proximité comprend en outre un second fragment d'anticorps ayant une affinité pour le second épitope.

11. Procédé de détection d'une protéine cible dans un échantillon, le procédé comprenant :
la formation d'un complexe protéique comprenant une protéine cible, un premier oligonucléotide et un deuxième oligonucléotide ;
la production d'un acide nucléique matrice à partir des premier et deuxième oligonucléotides ;
l'amplification de l'acide nucléique matrice, l'amplification générant un acide nucléique amplifié ;
le marquage de l'acide nucléique amplifié avec une sonde de détection qui s'hybride à l'acide nucléique amplifié, le marquage générant un acide nucléique marqué ;
la mise en contact de l'acide nucléique marqué avec un substrat d'écoulement latéral, le substrat d'écoulement latéral comprenant une sonde de capture configurée pour s'hybrider à l'acide nucléique marqué ; et
la détection d'un signal provenant du substrat d'écoulement latéral en réponse à l'hybridation de l'acide nucléique marqué avec la sonde de capture.

12. Procédé selon la revendication 11, dans lequel le substrat d'écoulement latéral fait partie d'un dispositif fluidique.

13. Procédé selon la revendication 12, dans lequel la formation du complexe protéique, la production de l'acide nucléique matrice et l'amplification de l'acide nucléique matrice se déroulent dans une même chambre du dispositif fluidique, la chambre étant en amont du substrat d'écoulement latéral.

14. Procédé selon la revendication 12, dans lequel le marquage se déroule à un emplacement du dispositif fluidique qui intègre le substrat d'écoulement latéral.

15. Procédé selon la revendication 12, dans lequel la détection comprend la détection optique du substrat d'écoulement latéral à l'aide d'un capteur.
